# EUROPEAN PATENT APPLICATION

(11) **EP 4 603 494 A1**
(43) Date of publication of application: **20.08.2025**
(21) Application number: 23876728.9
(22) Date of filing: 11.10.2023
(51) Int. Cl.: C07F 15/00, C07F 15/04, C08F 4/70

(54) **PHOSPHINE-PHENOL LATE TRANSITION METAL COMPLEX, AND PREPARATION METHOD THEREFOR AND USE THEREOF**

(30) Priority: 11.10.2022 CN 202211242003
(71) Applicant: CHINA PETROLEUM & CHEMICAL CORPORATION, Chaoyang District Beijing 100728 (CN); Sinopec (Beijing) Research Institute of Chemical Industry Co., Ltd., Beijing 100013 (CN)
(72) Inventor: GAO, Rong, Beijing 100013 (CN); GOU, Qingqiang, Beijing 100013 (CN); GUO, Zifang, Beijing 100013 (CN); ZHANG, Randi, Beijing 100013 (CN); LAI, Jingjing, Beijing 100013 (CN); WANG, Ying, Beijing 100013 (CN); AN, Jingyan, Beijing 100013 (CN); YUE, Qiang, Beijing 100013 (CN); SONG, Zhihui, Beijing 100013 (CN); LI, Xinyang, Beijing 100013 (CN)
(74) Representative: Hoffmann Eitle
(86) International application number: PCT/CN2023/123997
(87) International publication number: WO 2024/078527

(57) **Abstract**

The present invention relates to the technical field of olefin polymerization catalysts, and discloses a phosphine-phenol late transition metal complex and preparation method therefor and use thereof. The structural formula of the phosphine-phenol late transition metal complex is shown in formula (I), wherein, R₁ is selected from substituted or unsubstituted C1-C20 hydrocarbyl, R₃ and R₄ are each independently selected from halogen, C1-C10 hydrocarbyl, -P(R₅)₃, -NR₆R₇ and -OR₈R₉, wherein R₅ is substituted or unsubstituted C1-C10 alkyl or aryl, R₆, R₇, R₈ and R₉ are each independently selected from C1-C10 hydrocarbyl, and R₆ and R₇, as well as R₈ and R₉, are optionally interconnected to form a five- or six-membered ring. Under similar polymerization conditions, by using the metal complex of the present invention as a catalyst for olefin polymerization, a higher homopolymerization/copolymerization activity can be achieved, and the obtained polymer has an obviously higher molecular weight and a narrower molecular weight distribution.

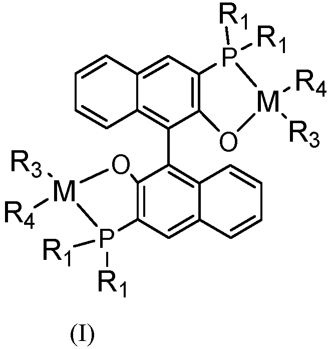

## Description

### Technical field

The present invention relates to the technical field of olefin polymerization catalysts, and particularly relates to a phosphine-phenol late transition metal complex and preparation method therefor and use thereof.

### Background art

Polyolefin resins have excellent environmental coordination compared with other resin materials, and thus are widely used in industry and life. Polyethylene resin is an important polyolefin resin. Industrialized polyethylene catalysts include Ziegler-Natta type catalysts (see, for example, DE Pat 889229 (1953); IT Pat 545332 (1956) and IT Pat 536899 (1955); Chem. Rev., 2000, 100, 1169 and related literatures of the Special Edition), Phillips type catalysts (see, for example, Belg. Pat. 530617 (1955); Chem. Rev. 1996, 96, 3327) and metallocene catalysts (see, for example, W. Kaminsky, Metalorganic Catalysts for Synthesis and Polymerization, Berlin: Springer, 1999), as well as the late transition metal complexes-type highly efficient ethylene oligomerization and polymerization catalysts developed rapidly in recent years. For example, in 1995, Brookhart et al. reported a type of α-diimine Ni (II) complexes that can polymerize ethylene with high activity. However, due to obvious "chain walking" of this type of catalyst, the resulting polymer has a high degree of branching. In addition, phosphine sulfonic acid neutral palladium complex is another classic catalyst for ethylene homopolymerization/copolymerization, which has received widespread attention since its first report by Pugh's research group in 2002. Mecking, Nozaki, Jordon and other research groups have conducted in-depth studies on the copolymerization reaction behavior and catalytic mechanism of ethylene initiated by this type of catalyst, further promoting the development of late transition metal catalysts.

The existing ethylene gas-phase polymerization process usually requires a polymerization temperature of 85°C or higher, and ethylene solution polymerization process usually requires a polymerization temperature of 130-250°C. Therefore, it is necessary to develop a late transition metal catalyst suitable for use at higher temperatures such as 80°C or higher to meet the requirements of existing gas-phase, solution ethylene polymerization devices.

### Contents of the invention

An object of the present invention is to provide a phosphine-phenol late transition metal complex and preparation method therefor and use thereof, the phosphine-phenol late transition metal complex has good thermal stability, particularly, the metal complex can still maintain a higher ethylene polymerization activity at a higher temperature, and the obtained polymer has a narrower molecular weight distribution.

In order to achieve the above object, in a first aspect, the present invention provides a phosphine-phenol late transition metal complex, the structural formula of which is shown in formula (I), 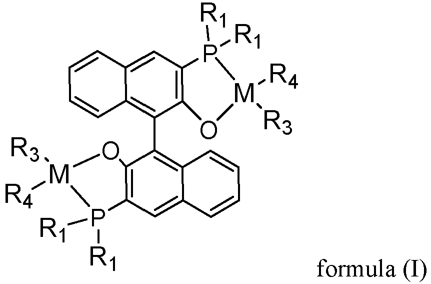 wherein, M is selected from Group VIII metals, R₁ is selected from substituted or unsubstituted C6-C20 aryl, R₃ and R₄ are each independently selected from halogen, C1-C10 hydrocarbyl, P(R₅)₃, NR₆R₇ and OR₈R₉, or R₃ and R₄ are interconnected to form an eight-membered ring, wherein R₅ is selected from substituted or unsubstituted C1-C10 alkyl and substituted or unsubstituted C6-C10 aryl, R₆, R₇, R₈ and R₉ are each independently selected from C1-C10 hydrocarbyl, or R₆, R₇ and N are interconnected to form a five- or six-membered ring, or R₈, R₉ and O are interconnected to form a five- or six-membered ring.

Preferably, R₅ includes C1-C6 alkyl (such as methyl, ethyl, propyl or a combination thereof), C6-C10 aryl (such as phenyl, phenylmethyl, phenylethyl or a combination thereof) or a combination thereof.

Preferably, C6-C20 aryl (including C6-C15 aryl) is selected from phenyl, 4-methyl phenyl, 4-ethyl phenyl, dimethyl phenyl, vinyl phenyl, anthryl, naphthyl, or biphenyl.

Preferably, for substituted C6-C20 aryl, the substituent thereof is selected from H, halogen, hydroxy, substituted or unsubstituted alkoxy (preferably C1-C6 alkoxy), substituted or unsubstituted C1-C20 hydrocarbyl.

Preferably, for substituted alkoxy and substituted C1-C20 hydrocarbyl, the substituents thereof are selected from halogen, hydroxy, C1-C6 alkyl, halo-substituted C1-C6 alkyl, C1-C6 alkoxy and halo-substituted C1-C6 alkoxy.

Preferably, the halo is selected from fluoro, chloro, bromo, or iodo.

Preferably, the halo includes monohalo, dihalo or perhalo, such as monofluoro, difluoro or perfluoro.

Preferably, C1-C10 hydrocarbyl includes C1-C8 hydrocarbyl (such as C1-C6 alkyl) or C7-C10 aralkyl, the aralkyl including, but not limited to: phenylmethyl, phenylethyl, phenyl n-propyl, phenyl isopropyl, phenyl n-butyl and phenyl tert-butyl.

Preferably, C1-C20 hydrocarbyl includes C1-C8 hydrocarbyl (such as C1-C6 alkyl), preferably methyl, ethyl or propyl; and/or C6-C15 aryl.

Preferably, the C1-C6 alkyl is selected from methyl, ethyl, n-propyl, isopropyl, n-butyl, isobutyl, n-pentyl, isopentyl, n-hexyl, isohexyl and 3,3-dimethyl butyl.

Preferably, the C1-C6 alkoxy is selected from methoxy, ethoxy, n-propoxy, isopropoxy, n-butoxy, isobutoxy, n-pentyloxy, isopentyloxy, n-hexyloxy, isohexyloxy and 3,3-dimethyl butoxy.

Preferably, the halogen is selected from fluorine, chlorine, bromine and iodine.

Unless otherwise specified, the above definitions of groups also apply to the definitions of groups in other preferred or related structures described in the context.

Preferably, the structural formulaof the phosphine-phenol late transition metal complex is shown in formula (II), 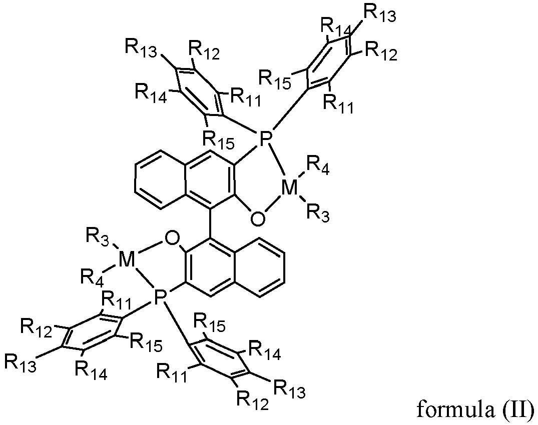 wherein, R₁₁-R₁₅ are each independently selected from H, halogen, hydroxy, substituted or unsubstituted alkoxy or substituted or unsubstituted C1-C20 hydrocarbyl; preferably, for substituted alkoxy and substituted C1-C20 hydrocarbyl, the substituents thereof are selected from halogen, hydroxy, C1-C6 alkyl, halo-substituted C1-C6 alkyl, C1-C6 alkoxy and halo-substituted C1-C6 alkoxy.

Preferably, M is selected from nickel and palladium.

Preferably, R₁₁-R₁₅ are each independently selected from H, halogen, hydroxy, substituted or unsubstituted alkoxy or substituted or unsubstituted C1-C10 alkyl and substituted or unsubstituted C6-C15 aryl.

Preferably, at least one of R₃ and R₄ is selected from halogen and C1-C8 hydrocarbyl, or R₃ and R₄ are interconnected to form an eight-membered ring.

Preferably, for substituted alkoxy and substituted C1-C10 alkyl and substituted C6-C15 aryl, the substituents are independently selected from halogen, hydroxy, C1-C6 alkyl, halo-substituted C1-C6 alkyl, C1-C6 alkoxy and halo-substituted C1-C6 alkoxy.

Preferably, the halo is selected from fluoro, chloro, bromo, or iodo.

Preferably, the halo includes monohalo, dihalo or perhalo. Preferably, the C1-C6 alkyl is selected from methyl, ethyl, n-propyl, isopropyl, n-butyl, isobutyl, n-pentyl, isopentyl, n-hexyl, isohexyl and 3,3-dimethyl butyl.

Preferably, the C1-C6 alkoxy is selected from methoxy, ethoxy, n-propoxy, isopropoxy, n-butoxy, isobutoxy, n-pentyloxy, isopentyloxy, n-hexyloxy, isohexyloxy and 3,3-dimethyl butoxy.

Preferably, the halogen is selected from fluorine, chlorine, bromine and iodine. In a second aspect, the present invention provides a method for preparing the above-mentioned phosphine-phenol late transition metal complex, the method comprising:
(1) reacting a compound of formula (III) with a compound of formula (IV), to obtain a ligand;
(2) reacting the ligand with an M metal compound; 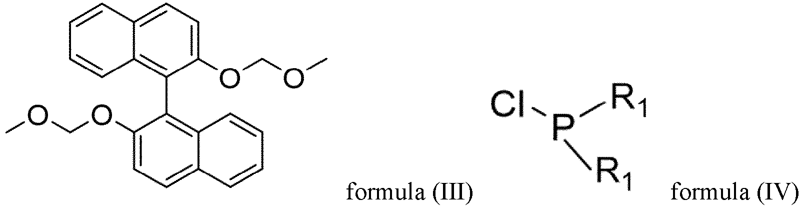 wherein, M metal is selected from Group VIII metals, preferably nickel and/or palladium; the definition of R₁ is the same as described above.

Preferably, the M metal compound is selected from at least one of dimethyl dipyridinyl nickel, bis(1,5-cyclooctadienyl) nickel, tetrapyridinyl nickel dichloride, ethylene glycol dimethyl ether nickel bromide, ethylene glycol dimethyl ether nickel chloride, di(trimethylphosphino) nickel dichloride, di(pyridinyl) di[(trimethylsilyl)methyl] nickel, (phenyl)(N,N,N',N'-tetramethyl-1,2-ethylene diamine) nickel chloride, dibenzyl dipyridinyl nickel, phenyl (trimethylphosphino) nickel bromide, phenyl (triethylphosphino) nickel chloride, diphenyl di(trimethylphosphino) nickel, di(trimethylphosphino) nickel dichloride, dimethyl dipyridinyl palladium, dipyridinyl palladium dichloride, di(pyridinyl) di[(trimethylsilyl)methyl] palladium, dibenzyl dipyridinyl palladium and methyl-1,5-cyclooctadiene-palladium chloride.

Preferably, the reaction of step (2) is carried out in the presence of a reaction solvent, the reaction solvent being tetrahydrofuran.

In a third aspect, the present invention provides use of the above-mentioned phosphine-phenol late transition metal complex in olefin polymerization.

In a fourth aspect, the present invention provides an olefin polymerization method, comprising carrying out olefin polymerization reaction in the presence of the above-mentioned phosphine-phenol late transition metal complex.

Preferably, the olefin polymerization reaction has a temperature of -78°C~200°C, preferably -20°C~150°C, and a pressure of 0.01~10MPa, preferably 0.01~5MPa.

The phosphine-phenol late transition metal complex, when used as an olefin polymerization catalyst, has higher homopolymerization/copolymerization activity, and can catalyze olefin polymerization at a higher temperature. The obtained olefin polymer has an obviously higher molecular weight.

Compared with the prior art, the technical solution of the present invention has the following advantages:
(1) The presently described synthesis method of the phosphine-phenol late transition metal complex is simple and easy to perform.
(2) The presently described phosphine-phenol late transition metal complex can catalyze the polymerization of ethylene with high activity, especially maintaining a higher polymerization activity at a higher polymerization temperature.
(3) The presently described phosphine-phenol late transition metal complex as an olefin polymerization catalyst exhibits higher performance for copolymerization of ethylene with α-olefins or polar monomers.

### Detailed description of specific embodiments

The specific embodiments of the present invention are described in detail below. It should be understood that the specific embodiments described herein are only for illustrating and explaining the present invention, and are not intended to limit the present invention.

The endpoints of the ranges and any values disclosed herein are not limited to the exact ranges or values, and these ranges or values should be understood to encompass values close to these ranges or values. For numerical ranges, the value between the endpoint values of each range, the value between the endpoint values of each range and individual point values, and the value between individual point values can be combined with each other to obtain one or more new numerical ranges, which should be considered as having been specifically disclosed herein. The structural formulaof the phosphine-phenol late transition metal complex according to the present invention is shown in formula (I), 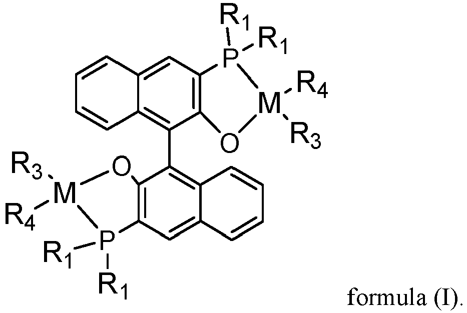 wherein, M is selected from Group VIII metals, R₁ is selected from substituted or unsubstituted C6-C20 aryl, R₃ and R₄ are each independently selected from halogen, C1-C10 hydrocarbyl, P(R₅)₃, NR₆R₇ and OR₈R₉, or R₃ and R₄ are interconnected to form an eight-membered ring, wherein R₅ is selected from substituted or unsubstituted C1-C10 alkyl and substituted or unsubstituted C6-C10 aryl, R₆, R₇, R₈ and R₉ are each independently selected from C1-C10 hydrocarbyl, or R₆, R₇ and N are interconnected to form a five- or six-membered ring, or R₈, R₉ and O are interconnected to form a five- or six-membered ring.

In a more preferred embodiment, the structural formula of the phosphine-phenol late transition metal complex is shown in formula (II), 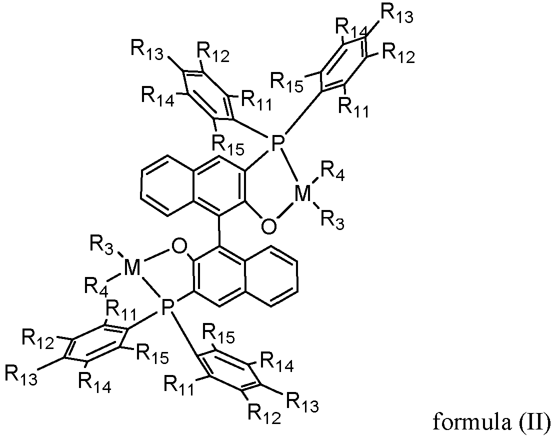 wherein, R₁₁-R₁₅ are each independently selected from H, halogen, hydroxy, substituted or unsubstituted alkoxy or substituted or unsubstituted C1-C20 hydrocarbyl. Preferably, R₁₁-R₁₅ are each independently selected from H, halogen, hydroxy, substituted or unsubstituted C1-C6 alkoxy or substituted or unsubstituted C1-C10 alkyl and substituted or unsubstituted C6-C15 aryl.

In formula (I) and formula (II), in a preferred case, M is selected from nickel and palladium.

In formula (I) and formula (II), in a preferred case, at least one of R₃ and R₄ is selected from halogen and C1-C8 hydrocarbyl, or R₃ and R₄ are interconnected to form an eight-membered ring. In one embodiment, R₃ and R₄ are each independently selected from halogen and C1-C8 hydrocarbyl (such as C1-C8 alkyl or aryl). In another embodiment, R₃ is selected from halogen and C1-C8 hydrocarbyl (such as C1-C8 alkyl or aryl), R₄ is selected from P(R₅)₃, NR₆R₇ and OR₈R₉, wherein R₅ is selected from substituted or unsubstituted C1-C10 alkyl and substituted or unsubstituted C6-C10 aryl, R₆, R₇, R₈ and R₉ are each selected from C1-C6 hydrocarbyl, and R₆, R₇ and N are interconnected to form a five- or six-membered ring, R₈, R₉ and O are interconnected to form a five- or six-membered ring. In still another embodiment, R₃ and R₄ are interconnected to form an eight-membered ring.

In the present invention, when R₃ and R₄ are interconnected to form an eight-membered ring, R₃, R₄ may be interconnected to form cyclooctenyl and the like. When R₃ and/or R₄ are each independently selected from P(R₅)₃, they may be PMe₃, PPh₃, PEt₃ and the like for example. When R₃ and/or R₄ are each independently selected from NR₆R₇, they may be pyridine (C₅H₅N), 2-methyl pyridine, 3-methyl pyridine, 4-methyl pyridine and the like for example. When R₃ and/or R₄ are each independently selected from OR₈R₉, it may be tetrahydrofuranyl (C₄H₈O), 2-methyl tetrahydrofuranyl, 3-methyl tetrahydrofuranyl, 2,5-dimethyl tetrahydrofuranyl, 2,2-dimethyl tetrahydrofuran and the like for example. Herein, "Me" refers to methyl, "Ph" refers to phenyl, "Et" refers to ethyl.

In the present invention, the term "substituted" in the expression "substituted or unsubstituted" refers to containing substituent(s), and the substituent(s) herein may be independently selected from halogen, hydroxy, C1-C6 alkyl, halo-substituted C1-C6 alkyl, C1-C6 alkoxy and halo-substituted C1-C6 alkoxy. Preferably, the halo is selected from fluoro, chloro, bromo, or iodo.

In the present invention, alkyl (such as C1-C6 alkyl, C1-C8 alkyl or C1-C10 alkyl) may be independently selected from methyl, ethyl, n-propyl, isopropyl, n-butyl, isobutyl, n-pentyl, isopentyl, n-hexyl, isohexyl and 3,3-dimethyl butyl.

In the present invention, alkoxy (such as C1-C6 alkoxy) may be independently selected from methoxy, ethoxy, n-propoxy, isopropoxy, n-butoxy, isobutoxy, n-pentyloxy, isopentyloxy, n-hexyloxy, isohexyloxy and 3,3-dimethyl butoxy.

In the present invention, aryl (such as C6-C10 aryl or C6-C15 aryl) may be independently selected from phenyl, 4-methyl phenyl, 4-ethyl phenyl, dimethyl phenyl, vinyl phenyl, anthryl, naphthyl, or biphenyl and the like.

In the present invention, the halogen is independently selected from fluorine, chlorine, bromine and iodine.

In a further preferred embodiment, the phosphine-phenol late transition metal complex is selected from a group consisting of the following complexes,
complex 1: complex of formula (II), wherein M is Ni, R₁₁-R₁₅ are H, R₃ is methyl, R₄ is pyridinyl (at this time, the N on pyridinyl coordinates with the metal Ni);
complex 2: complex of formula (II), wherein M is Ni, R₁₁-R₁₅ are F, R₃ is methyl, R₄ is pyridinyl;
complex 3: complex of formula (II), wherein M is Ni, R₁₁ is phenyl, R₁₂-R₁₅ are H, R₃ is methyl, R₄ is pyridinyl;
complex 4: complex of formula (II), wherein M is Ni, R₁₁ is methyl, R₁₂-R₁₅ are H, R₃ is methyl, R₄ is pyridinyl;
complex 5: complex of formula (II), wherein M is Ni, R₁₃ is methyl, R₁₁, R₁₂, R₁₄ and R₁₅ are H, R₃ is methyl, R₄ is pyridinyl;
complex 6: complex of formula (II), wherein M is Ni, R₁₃ is -CF₃, R₁₁, R₁₂, R₁₄ and R₁₅ are H, R₃ is methyl, R₄ is pyridinyl;
complex 7: complex of formula (II), wherein M is Ni, R₁₃ is F, R₁₁, R₁₂, R₁₄ and R₁₅ are H, R₃ is methyl, R₄ is pyridinyl;
complex 8: complex of formula (II), wherein M is Ni, R₁₃ is Cl, R₁₁, R₁₂, R₁₄ and R₁₅ are H, R₃ is methyl, R₄ is pyridinyl;
complex 9: complex of formula (II), wherein M is Ni, R₁₁, R₁₃ and R₁₅ are methyl, R₁₂ and R₁₄ are H, R₃ is methyl, R₄ is pyridinyl;
complex 10: complex of formula (II), wherein M is Ni, R₁₃ is methoxy, R₁₁, R₁₂, R₁₄ and R₁₅ are H, R₃ is methyl, R₄ is pyridinyl;
complex 11: complex of formula (II), wherein M is Ni, R₁₂ and R₁₄ are methyl, R₁₁, R₁₃ and R₁₅ are H, R₃ is methyl, R₄ is pyridinyl;
complex 12: complex of formula (II), wherein M is Ni, R₁₂ and R₁₄ are methyl, R₁₃ is methoxy, R₁₁ and R₁₅ are H, R₃ is methyl, R₄ is pyridinyl;
complex 13: complex of formula (II), wherein M is Ni, R₁₂ and R₁₄ are -CF₃, R₁₁, R₁₃ and R₁₅ are H, R₃ is methyl, R₄ is pyridinyl;
complex 14: complex of formula (II), wherein M is Ni, R₁₁ and R₁₅ are methoxy, R₁₂-R₁₄ are H, R₃ is methyl, R₄ is pyridinyl;
complex 15: complex of formula (II), wherein M is Ni, R₁₁ is methoxy, R₁₂-R₁₅ are H, R₃ is methyl, R₄ is pyridinyl;
complex 16: complex of formula (II), wherein M is Ni, R₁₁-R₁₅ are H, R₃ is phenyl, R₄ is PMe₃;
complex 17: complex of formula (II), wherein M is Ni, R₁₁ and R₁₅ are F, R₃ is phenyl, R₄ is PMe₃;
complex 18: complex of formula (II), wherein M is Ni, R₁₁ is phenyl, R₁₂-R₁₅ are H, R₃ is phenyl, R₄ is PMe₃;
complex 19: complex of formula (II), wherein M is Ni, R₁₁ is methyl, R₁₂-R₁₅ are H, R₃ is phenyl, R₄ is PMe₃;
complex 20: complex of formula (II), wherein M is Ni, R₁₃ is methyl, R₁₁, R₁₂, R₁₄ and R₁₅ are H, R₃ is phenyl, R₄ is PMe₃;
complex 21: complex of formula (II), wherein M is Ni, R₁₃ is -CF₃, R₁₁, R₁₂, R₁₄ and R₁₅ are H, R₃ is phenyl, R₄ is PMe₃;
complex 22: complex of formula (II), wherein M is Ni, R₁₃ is F, R₁₁, R₁₂, R₁₄ and R₁₅ are H, R₃ is phenyl, R₄ is PMe₃;
complex 23: complex of formula (II), wherein M is Ni, R₁₃ is Cl, R₁₁, R₁₂, R₁₄ and R₁₅ are H, R₃ is phenyl, R₄ is PMe₃;
complex 24: complex of formula (II), wherein M is Ni, R₁₁, R₁₃ and R₁₅ are methyl, R₁₂ and R₁₄ are H, R₃ is phenyl, R₄ is PMe₃;
complex 25: complex of formula (II), wherein M is Ni, R₁₃ is methoxy, R₁₁, R₁₂, R₁₄ and R₁₅ are H, R₃ is phenyl, R₄ is PMe₃;
complex 26: complex of formula (II), wherein M is Ni, R₁₂ and R₁₄ are methyl, R₁₁, R₁₃ and R₁₅ are H, R₃ is phenyl, R₄ is PMe₃;
complex 27: complex of formula (II), wherein M is Ni, R₁₂ and R₁₄ are methyl, R₁₃ is methoxy, R₁₁ and R₁₅ are H, R₃ is phenyl, R₄ is PMe₃;
complex 28: complex of formula (II), wherein M is Ni, R₁₂ and R₁₄ are -CF₃, R₁₁, R₁₃ and R₁₅ are H, R₃ is phenyl, R₄ is PMe₃;
complex 29: complex of formula (II), wherein M is Ni, R₁₁ and R₁₅ are methoxy, R₁₂-R₁₄ are H, R₃ is phenyl, R₄ is PMe₃;
complex 30: complex of formula (II), wherein M is Ni, R₁₁ is methoxy, R₁₂-R₁₅ are H, R₃ is phenyl, R₄ is PMe₃;
complex 31: complex of formula (II), wherein M is Ni, R₁₁-R₁₅ are H, R₃, R₄ are interconnected to form cyclooctenyl;
complex 32: complex of formula (II), wherein M is Ni, R₁₁ is phenyl, R₁₂-R₁₅ are H, R₃, R₄ are interconnected to form cyclooctenyl;
complex 33: complex of formula (II), wherein M is Ni, R₁₁ is phenyl, R₁₂-R₁₅ are H, R₃, R₄ are interconnected to form cyclooctenyl;
complex 34: complex of formula (II), wherein M is Ni, R₁₃ is methoxy, R₁₁, R₁₂, R₁₄ and R₁₅ are H, R₃, R₄ are interconnected to form cyclooctenyl;
complex 35: complex of formula (II), wherein M is Ni, R₁₂ and R₁₄ are -CF₃, R₁₁, R₁₃ and R₁₅ are H, R₃, R₄ are interconnected to form cyclooctenyl;
complex 36: complex of formula (II), wherein M is Ni, R₁₁ and R₁₅ are methoxy, R₁₂-R₁₄ are H, R₃, R₄ are interconnected to form cyclooctenyl;
complex 37: complex of formula (II), wherein M is Ni, R₁₁ is methoxy, R₁₂-R₁₅ are H, R₃, R₄ are interconnected to form cyclooctenyl;
complex 38: complex of formula (II), wherein M is Ni, R₁₁-R₁₅ are H, R₃ is Cl, R₄ is tetrahydrofuranyl (at this time, the O on tetrahydrofuranyl coordinates with the metal Ni);
complex 39: complex of formula (II), wherein M is Ni, R₁₁-R₁₅ are F, R₃ is Cl, R₄ is tetrahydrofuranyl;
complex 40: complex of formula (II), wherein M is Ni, R₁₁ is phenyl, R₁₂-R₁₅ are H, R₃ is Cl, R₄ is tetrahydrofuranyl;
complex 41: complex of formula (II), wherein M is Ni, R₁₁ is methyl, R₁₂-R₁₅ are H, R₃ is Cl, R₄ is tetrahydrofuranyl;
complex 42: complex of formula (II), wherein M is Ni, R₁₃ is methyl, R₁₁, R₁₂, R₁₄ and R₁₅ are H, R₃ is Cl, R₄ is tetrahydrofuranyl;
complex 43: complex of formula (II), wherein M is Ni, R₁₃ is -CF₃, R₁₁, R₁₂, R₁₄ and R₁₅ are H, R₃ is Cl, R₄ is tetrahydrofuranyl;
complex 44: complex of formula (II), wherein M is Ni, R₁₃ is F, R₁₁, R₁₂, R₁₄ and R₁₅ are H, R₃ is Cl, R₄ is tetrahydrofuranyl;
complex 45: complex of formula (II), wherein M is Ni, R₁₃ is Cl, R₁₁, R₁₂, R₁₄ and R₁₅ are H, R₃ is Cl, R₄ is tetrahydrofuranyl;
complex 46: complex of formula (II), wherein M is Ni, R₁₁, R₁₃ and R₁₅ are methyl, R₁₂ and R₁₄ are H, R₃ is Cl, R₄ is tetrahydrofuranyl;
complex 47: complex of formula (II), wherein M is Ni, R₁₃ is methoxy, R₁₁, R₁₂, R₁₄ and R₁₅ are H, R₃ is Cl, R₄ is tetrahydrofuranyl;
complex 48: complex of formula (II), wherein M is Ni, R₁₂ and R₁₄ are methyl, R₁₁, R₁₃ and R₁₅ are H, R₃ is Cl, R₄ is tetrahydrofuranyl;
complex 49: complex of formula (II), wherein M is Ni, R₁₂ and R₁₄ are methyl, R₁₃ is methoxy, R₁₁ and R₁₅ are H, R₃ is Cl, R₄ is tetrahydrofuranyl;
complex 50: complex of formula (II), wherein M is Ni, R₁₂ and R₁₄ are -CF₃, R₁₁, R₁₃ and R₁₅ are H, R₃ is Cl, R₄ is tetrahydrofuranyl;
complex 51: complex of formula (II), wherein M is Ni, R₁₁ and R₁₅ are methoxy, R₁₂-R₁₄ are H, R₃ is Cl, R₄ is tetrahydrofuranyl;
complex 52: complex of formula (II), wherein M is Ni, R₁₁ is methoxy, R₁₂-R₁₅ are H, R₃ is Cl, R₄ is tetrahydrofuranyl;
complex 53: complex of formula (II), wherein M is Ni, R₁₁-R₁₅ are H, R₃ is Br, R₄ is tetrahydrofuranyl;
complex 54: complex of formula (II), wherein M is Ni, R₁₁-R₁₅ are F, R₃ is Br, R₄ is tetrahydrofuranyl;
complex 55: complex of formula (II), wherein M is Ni, R₁₁ is phenyl, R₁₂-R₁₅ are H, R₃ is Br, R₄ is tetrahydrofuranyl;
complex 56: complex of formula (II), wherein M is Ni, R₁₁ is methyl, R₁₂-R₁₅ are H, R₃ is Br, R₄ is tetrahydrofuranyl;
complex 57: complex of formula (II), wherein M is Ni, R₁₃ is methyl, R₁₁, R₁₂, R₁₄ and R₁₅ are H, R₃ is Br, R₄ is tetrahydrofuranyl;
complex 58: complex of formula (II), wherein M is Ni, R₁₃ is -CF₃, R₁₁, R₁₂, R₁₄ and R₁₅ are H, R₃ is Br, R₄ is tetrahydrofuranyl;
complex 59: complex of formula (II), wherein M is Ni, R₁₃ is F, R₁₁, R₁₂, R₁₄ and R₁₅ are H, R₃ is Br, R₄ is tetrahydrofuranyl;
complex 60: complex of formula (II), wherein M is Ni, R₁₃ is Cl, R₁₁, R₁₂, R₁₄ and R₁₅ are H, R₃ is Br, R₄ is tetrahydrofuranyl;
complex 61: complex of formula (II), wherein M is Ni, R₁₁, R₁₃ and R₁₅ are methyl, R₁₂ and R₁₄ are H, R₃ is Br, R₄ is tetrahydrofuranyl;
complex 62: complex of formula (II), wherein M is Ni, R₁₃ is methoxy, R₁₁, R₁₂, R₁₄ and R₁₅ are H, R₃ is Br, R₄ is tetrahydrofuranyl;
complex 63: complex of formula (II), wherein M is Ni, R₁₂ and R₁₄ are methyl, R₁₁, R₁₃ and R₁₅ are H, R₃ is Br, R₄ is tetrahydrofuranyl;
complex 64: complex of formula (II), wherein M is Ni, R₁₂ and R₁₄ are methyl, R₁₃ is methoxy, R₁₁ and R₁₅ are H, R₃ is Br, R₄ is tetrahydrofuranyl;
complex 65: complex of formula (II), wherein M is Ni, R₁₂ and R₁₄ are -CF₃, R₁₁, R₁₃ and R₁₅ are H, R₃ is Br, R₄ is tetrahydrofuranyl;
complex 66: complex of formula (II), wherein M is Ni, R₁₁ and R₁₅ are methoxy, R₁₂-R₁₄ are H, R₃ is Br, R₄ is tetrahydrofuranyl;
complex 67: complex of formula (II), wherein M is Ni, R₁₁ is methoxy, R₁₂-R₁₅ are H, R₃ is Br, R₄ is tetrahydrofuranyl;
complex 68: complex of formula (II), wherein M is Pd, R₁₁-R₁₅ are H, R₃ is methyl, R₄ is pyridinyl;
complex 69: complex of formula (II), wherein M is Pd, R₁₁-R₁₅ are F, R₃ is methyl, R₄ is pyridinyl;
complex 70: complex of formula (II), wherein M is Pd, R₁₁ is phenyl, R₁₂-R₁₅ are H, R₃ is methyl, R₄ is pyridinyl;
complex 71: complex of formula (II), wherein M is Pd, R₁₁ is phenyl, R₁₂-R₁₅ are H, R₃ is methyl, R₄ is pyridinyl;
complex 72: complex of formula (II), wherein M is Pd, R₁₁ is methyl, R₁₂-R₁₅ are H, R₃ is methyl, R₄ is pyridinyl;
complex 73: complex of formula (II), wherein M is Pd, R₁₃ is methyl, R₁₁, R₁₂, R₁₄ and R₁₅ are H, R₃ is methyl, R₄ is pyridinyl;
complex 74: complex of formula (II), wherein M is Pd, R₁₃ is -CF₃, R₁₁, R₁₂, R₁₄ and R₁₅ are H, R₃ is methyl, R₄ is pyridinyl;
complex 75: complex of formula (II), wherein M is Pd, R₁₃ is F, R₁₁, R₁₂, R₁₄ and R₁₅ are H, R₃ is methyl, R₄ is pyridinyl;
complex 76: complex of formula (II), wherein M is Pd, R₁₃ is Cl, R₁₁, R₁₂, R₁₄ and R₁₅ are H, R₃ is methyl, R₄ is pyridinyl;
complex 77: complex of formula (II), wherein M is Pd, R₁₁, R₁₃ and R₁₅ are methyl, R₁₂ and R₁₄ are H, R₃ is methyl, R₄ is pyridinyl;
complex 78: complex of formula (II), wherein M is Pd, R₁₃ is methoxy, R₁₁, R₁₂, R₁₄ and R₁₅ are H, R₃ is methyl, R₄ is pyridinyl;
complex 79: complex of formula (II), wherein M is Pd, R₁₂ and R₁₄ are methyl, R₁₁, R₁₃ and R₁₅ are H, R₃ is methyl, R₄ is pyridinyl;
complex 80: complex of formula (II), wherein M is Pd, R₁₂ and R₁₄ are methyl, R₁₃ is methoxy, R₁₁ and R₁₅ are H, R₃ is methyl, R₄ is pyridinyl;
complex 81: complex of formula (II), wherein M is Pd, R₁₂ and R₁₄ are -CF₃, R₁₁, R₁₃ and R₁₅ are H, R₃ is methyl, R₄ is pyridinyl;
complex 82: complex of formula (II), wherein M is Pd, R₁₁ and R₁₅ are methoxy, R₁₂-R₁₄ are H, R₃ is methyl, R₄ is pyridinyl;
complex 83: complex of formula (II), wherein M is Pd, R₁₁ is methoxy, R₁₂-R₁₅ are H, R₃ is methyl, R₄ is pyridinyl;
complex 84: complex of formula (II), wherein M is Pd, R₁₁-R₁₅ are H, R₃ is methyl, R₄ is Cl;
complex 85: complex of formula (II), wherein M is Pd, R₁₁ is phenyl, R₁₂-R₁₅ are H, R₃ is methyl, R₄ is Cl;
complex 86: complex of formula (II), wherein M is Pd, R₁₃ is methoxy, R₁₁, R₁₂, R₁₄ and R₁₅ are H, R₃ is methyl, R₄ is Cl;
complex 87: complex of formula (II), wherein M is Pd, R₁₂ and R₁₄ are -CF₃, R₁₁, R₁₃ and R₁₅ are H, R₃ is methyl, R₄ is Cl;
complex 88: complex of formula (II), wherein M is Pd, R₁₁ and R₁₅ are methoxy, R₁₂-R₁₄ are H, R₃ is methyl, R₄ is Cl;
complex 89: complex of formula (II), wherein M is Pd, R₁₁ is methoxy, R₁₂-R₁₅ are H, R₃ is methyl, R₄ is Cl.

A method for preparing the above-mentioned phosphine-phenol late transition metal complex may comprise:
(1) reacting a compound of formula (III) with a compound of formula (IV), to obtain a ligand;
(2) reacting the ligand with an M metal compound; 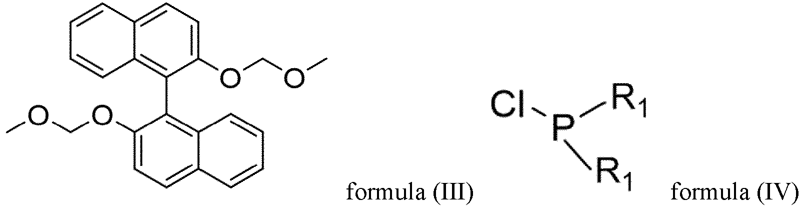
wherein, M metal is selected from Group VIII metals, preferably nickel and/or palladium; the definition of R₁ is the same as described above.

In the method of the present invention, the M metal compound may be selected from at least one of dimethyl dipyridinyl nickel (Py₂NiMe₂), bis(1,5-cyclooctadienyl) nickel (Ni(COD)₂), tetrapyridinyl nickel dichloride (Ni₂Cl₂Py₄), ethylene glycol dimethyl ether nickel bromide ((DME)NiBr₂), ethylene glycol dimethyl ether nickel chloride ((DME)NiCl₂), di(trimethylphosphino) nickel dichloride (NiCl₂(PMe₃)₂), di(pyridinyl) di[(trimethylsilyl)methyl] nickel (Ni(Py)₂(CH₂SiMe₃)₂), (phenyl)(N,N,N',N'-tetramethyl-1,2-ethylene diamine) nickel chloride (NiArBr(TMEDA)), dibenzyl dipyridinyl nickel (Ni(CH₂Ph)₂Py₂), phenyl (trimethylphosphino) nickel bromide (NiPhBr(PMe₃)₂, phenyl (triethylphosphino) nickel chloride (NiPhCl(PEt₃)₂), diphenyl di(trimethylphosphino) nickel (NiPh₂(PMe₃)₂), di(trimethylphosphino) nickel dichloride (NiCl₂(PMe₃)₂), dimethyl dipyridinyl palladium (Pd(Me)₂Py₂), dipyridinyl palladium dichloride (PdCl₂Py₂), di(pyridinyl) di[(trimethylsilyl)methyl] palladium (Pd(Py)₂(CH₂SiMe₃)₂), dibenzyl dipyridinyl palladium (Pd(CH₂Ph)₂Py₂) and methyl-1,5-cyclooctadiene-palladium chloride (Pd(COD)ClMe).

In the method of the present invention, the reaction of step (2) is carried out in the presence of a reaction solvent. In a specific embodiment, the reaction solvent may be selected from toluene and tetrahydrofuran. Preferably, the reaction solvent is tetrahydrofuran.

In a more preferred embodiment, the reaction process of step (1) and step (2) is shown in the following equation. 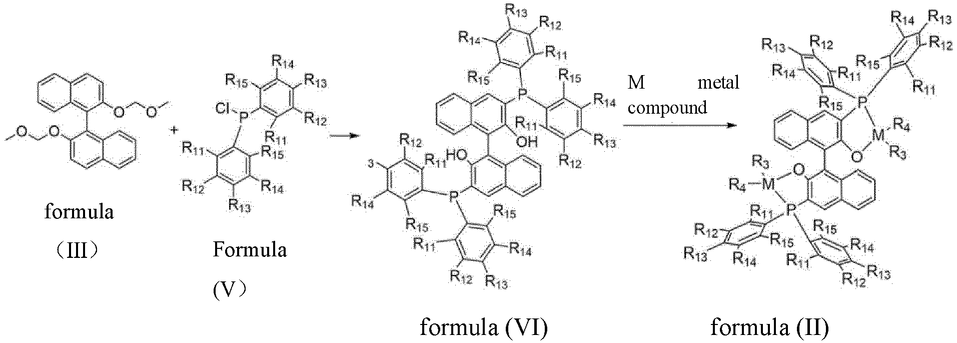 wherein, the definitions of M, R₃, R₄, R₁₁-R₁₅ are the same as described above.

In a specific embodiment, the preparation process of step (1) comprises: under a protective gas (such as nitrogen) atmosphere, dissolving a compound of formula (III) in anhydrous diethyl ether, adding a hydrogen abstracting agent (such as n-butyl lithium) at room temperature, stirring at room temperature, adding tetrahydrofuran, and further stirring the obtained black solution containing precipitate; next, adding a compound of formula (V), stirring at room temperature, and quenching with the addition of NH₄Cl aqueous solution; then extracting the organic phase with ethyl acetate, drying the obtained organic phase over anhydrous sodium sulfate, and recrystallizing with dichloromethane/hexane to obtain a yellow crystalline compound; afterwards, adding methanol and concentrated hydrochloric acid, reacting under reflux, after the completion of the reaction, removing the organic solvent, dissolving the product in ethyl acetate, neutralizing with NaHCO₃ aqueous solution, and extracting the organic phase, followed by drying, filtration, concentration, and column chromatography to obtain a ligand.

In a specific embodiment, the preparation process of step (2) comprises: under a protective gas (such as nitrogen) atmosphere, dissolving the ligand obtained in step (1) and the M metal compound in an organic solvent (such as toluene, tetrahydrofuran and the like), respectively, then mixing the solution of the ligand and the solution of the M metal compound with stirring and reacting at room temperature, filtering, concentrating the filtrate, and recrystallizing with heptane, to obtain the phosphine-phenol late transition metal complex of the present invention.

The present invention also provides use of the above-mentioned phosphine-phenol late transition metal complex in olefin polymerization. Preferably, the olefins include ethylene and α-olefins containing polar group.

The phosphine-phenol late transition metal complex of the present invention can be used as a catalyst for homopolymerization or copolymerization of olefins, and is particularly suitable for homopolymerization of ethylene or copolymerization of ethylene with other α-olefin and olefins having polar functional group such as hydroxy, carboxyl, ester group and the like, wherein the α-olefin is independently selected from at least one of propylene, butene, pentene, hexene, octene and 4-methyl-1-pentene. The olefin having polar functional group is a vinyl monomer containing one or more hydroxy, carboxyl, ester groups, and the vinyl monomer can contain a plurality of different polar groups in the same molecule.

In the present invention, another embodiment of the method for preparing the α-olefin polymer is to copolymerize (a) an α-olefin and (b) a (meth)acrylate monomer, a vinyl monomer or an allyl monomer in the presence of the above-mentioned phosphine-phenol late transition metal complex. The (meth)acrylate monomer in the present invention is represented by the general formula CH₂=C(R₃₁)CO₂(R₃₂), wherein R₃₁ may be H or C1-C10 hydrocarbyl, which may have a branched, cyclic, and/or unsaturated bond; R₃₂ may be C1-C30 hydrocarbyl, which may have a branched, cyclic, and/or unsaturated bond, and R₃₂ may contain a heteroatom at any position therein. When the number of carbon atoms of R₃₁ is 11 or more, it tends to show less polymerization activity. Accordingly, R₃₁ is H or C1-C10 hydrocarbyl. In a preferred case, R₃₁ is H or C1-C5 hydrocarbyl. More preferably, R₃₁ is H or methyl.

Similarly, when the number of carbon atoms of R₃₂ is more than 30, it tends to show less polymerization activity. Accordingly, R₃₂ has 1 to 30, preferably 1 to 12, and more preferably 1 to 8 carbon atoms.

Further, as the heteroatom optionally contained in R₃₂, oxygen, sulfur, selenium, phosphorus, nitrogen, silicon, fluorine, boron and the like can be selected. Among these heteroatoms, oxygen, silicon and fluorine are preferred, and oxygen is more preferred. Further, R₃₂ containing no heteroatom is also preferred.

Further preferably, specific examples of the (meth)acrylate monomer include, but are not limited to: methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, pentyl (meth)acrylate, hexyl (meth)acrylate, cyclohexyl (meth)acrylate, octyl (meth)acrylate, 2-ethylhexyl (meth)acrylate, nonyl (meth)acrylate, decyl (meth)acrylate, dodecyl (meth)acrylate, phenyl (meth)acrylate, toluyl (meth)acrylate, benzyl (meth)acrylate, hydroxyethyl (meth)acrylate, dimethylaminoethyl (meth)acrylate, diethylaminoethyl (meth)acrylate, 2-aminoethyl (meth)acrylate, 2-methoxyethyl (meth)acrylate, 3-methoxypropyl (meth)acrylate, glycidyl (meth)acrylate, oxiranyl (meth)acrylate, trifluoromethyl (meth)acrylate, 2-trifluoromethylethyl (meth)acrylate, perfluoroethyl (meth)acrylate, (meth)acrylamide, (meth)acryloyldimethylamide, 2-hydroxybutyl (meth)acrylate, 4-hydroxybutyl (meth)acrylate and the like. The above (meth)acrylate monomers may be used alone or in combination of two or more.

In the present invention, the olefins are C2-C16 olefins. Preferably, the olefins are selected from ethylene andα-olefins having 3 to 16 carbon atoms or cycloolefins. The cycloolefins include, but are not limited to: cyclobutene, cyclopentene, cyclohexene, cycloheptene, cyclooctene, norbornene and the like.

The present invention also provides an olefin polymerization method, comprising carrying out olefin polymerization reaction in the presence of the above-mentioned phosphine-phenol late transition metal complex. The olefin polymerization reaction may be homopolymerization or copolymerization.

The olefin polymerization reaction may have a temperature of -78°C~200°C, preferably -20°C ~150°C, and a pressure of 0.01~10MPa, preferably 0.01~5MPa. Here, the term "pressure" refers to ethylene pressure in the polymerization system, expressed in absolute pressure.

In the olefin polymerization method according to the present invention, the olefins are C2-C16 olefins.

According to an embodiment of the present invention, the olefins include C2-C16 α-olefins or cycloolefins.

According to an embodiment of the present invention, the olefins include ethylene.

According to an embodiment of the present invention, the olefins include ethylene and α-olefins or cycloolefins containing polar group.

According to an embodiment of the present invention, the olefin polymerization reaction is carried out by olefin monomers in a solvent, and the solvent for polymerization is selected from one or more of alkanes, aromatic hydrocarbons and halogenated hydrocarbons. Specifically, the solvent for polymerization is selected from one or more of hexane, pentane, heptane, benzene, toluene, dichloromethane, chloroform, chlorobenzene and dichloroethane, preferably one or more of hexane, toluene and heptane.

In the present invention, alkyl refers to linear alkyl, branched alkyl, or cyclic alkyl, including but not limited to: methyl, ethyl, n-propyl, isopropyl, n-butyl, sec-butyl, isobutyl, tert-butyl, n-pentyl, isopentyl, tert-pentyl, neopentyl, n-hexyl, n-heptyl, n-octyl, n-decyl, cyclopropyl, cyclopentyl, cyclohexyl, 4-methylcyclohexyl, 4-ethylcyclohexyl, 4-n-propylcyclohexyl, and 4-n-butylcyclohexyl.

In the present invention, alkenyl refers to linear alkenyl, branched alkenyl or cycloalkenyl, including but not limited to: vinyl, allyl, butenyl.

In the present invention, examples of aralkyl include, but are not limited to: phenylmethyl, phenylethyl, phenyl-n-propyl, phenyl-isopropyl, phenyl-n-butyl and phenyl-tert-butyl.

In the present invention, examples of alkaryl include, but are not limited to: tolyl, ethylphenyl, n-propylphenyl, isopropylphenyl, n-butylphenyl and tert-butylphenyl.

The phosphine-phenol late transition metal complex and preparation method therefor and use thereof according to the present invention are further illustrated by the following examples. The examples, which are implemented on the premise of the technical solution of the present invention, give detailed embodiments and specific operation processes. However, the protection scope of the present invention is not limited to the following examples.

The experimental methods in the following examples, unless otherwise specified, are conventional methods in the art. The experimental materials used in the following examples are commercially available unless otherwise specified.

The analytical characterization equipment and test methods used in the following Examples and Comparative Examples are as follows:
(1) Nuclear magnetic resonance instrument: Bruker DMX 300 (300 MHz), with tetramethylsilane (TMS) as the internal standard.
(2) Molecular weight and molecular weight distribution PDI (PDI = Mw/Mn) of the polymer: the measurement is carried out at 150 °C using a PL-GPC220 chromatograph with trichlorobenzene as the solvent (in which standard sample: PS, flow rate: 1.0 mL/min, chromatographic column: 3×PLgel 10um M1×ED-B, 300 ×7.5nm).
(3) Activity measurement method: the polymer is washed with hydrochloric acid-ethanol solution, dried in vacuum, and weighed. The polymerization activity is calculated as: polymer weight (g)/metal (mol) ×60/polymerization time (min).
(4) Analysis of the comonomer content of the polymer: the polymer sample is dissolved in 1,2,4-trichlorobenzene at 120°C, and analyzed by ¹H NMR, ¹³C NMR spectrum on a 400MHz Bruker Avance 400 nuclear magnetic resonance spectrometer using a 10mm PASEX 13 probe.

Example 1
complex 1: complex of formula (II), wherein M is Ni, R₁-R₁₅ are H, R₃ is methyl, R₄ is pyridinyl.

Under a nitrogen atmosphere, a compound of formula (III) (11.23g, 30mmol, S configuration) was dissolved in anhydrous diethyl ether (150mL), an n-butyl lithium solution (2.7M, 33.3mL, 90mmol) was added dropwise at room temperature, and the solution was stirred at room temperature for 4h. Tetrahydrofuran (150mL) was added, and the obtained black solution containing precipitate was further stirred for 1h. Diphenylphosphine chloride (PPh₂Cl) (90mmol, 16.7mL) was added at 0°C. The solution was stirred at room temperature for 1h, and then quenched with the addition of NH₄Cl aqueous solution (5mL). The organic phase was extracted with ethyl acetate, and the obtained organic phase was dried over anhydrous sodium sulfate, recrystallized with dichloromethane/hexane, to obtain a yellow crystalline compound (15.54 g). Methanol (100mL), 5mL of concentrated hydrochloric acid (37wt%) were added, and reacted under reflux for 16h. After the reaction as tracked using thin-layer chromatography (TLC) was completed, the organic solvent was removed, and the product was dissolved in ethyl acetate, and neutralized with NaHCO₃ aqueous solution. The organic phase was extracted, dried over anhydrous MgSO₄, filtered, concentrated, and separated by column chromatography (dichloromethane as solvent) to obtain ligand L₁, with a yield of 67 %. ¹H NMR (400 MHz, CDCl₃): δ = 5.41 (s, 2H), 7.13-7.15 (m, 2H), 7.24-7.29 (m, 4H), 7.38-7.45 (m, 22H), 7.62-7.64 (m, 2H). ³¹P NMR (162 MHz, CDCl₃): δ = -17.19 (s). High resolution mass spectrometry test: theoretical calculation value: 654.19; test value: 655.20.

Under a nitrogen atmosphere, the ligand L₁ (0.654g, 1mmol) and a metal nickel source (Py₂NiMe₂) (0.49g, 2mmol) were dissolved in toluene (10mL), respectively. Then, the solution of the ligand was added dropwise to the solution of the metal nickel source, and the reaction was carried out with stirring vigorously at room temperature for 10h, followed by filtration to obtain a brownish yellow solution. The solution was concentrated, and freeze-crystallized at -30°C to obtain naphthol-phosphine neutral nickel complex 1, with a yield of 76%. ¹H NMR (400 MHz, CDCl₃): δ: 7.92 (m, 8H), 7.58-7.63 (m, 2H), 7.32-7.50 (m, 22H), 7.22-7.34 (m, 4H), 7.10-7.15 (m, 2H), 6.60 (m, 2H), -0.41 (d, 6H). ³¹P NMR (162 MHz, CDCl₃) δ: 30.98. Elemental analysis test C₅₆H₄₆N₂Ni₂O₂P₂: theoretical calculation value: C, 70.19; H, 4.84; N, 2.92; test value: C, 69.94; H, 4.92; N, 2.84.

A 1L stainless steel polymerization kettle equipped with mechanical stirrer was dried continuously at 130°C for 6h, vacuumized while hot and purged 3 times with N₂. To the polymerization kettle, 500mL of toluene was charged, and 9.6mg (10µmol) of complex 1 was added. The reaction was carried out with stirring vigorously at 50°C for 20min while maintaining an ethylene pressure of 10 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

Example 2
complex 1: complex of formula (II), wherein M is Ni, R₁₁-R₁₅ are H, R₃ is methyl, R₄ is pyridinyl.

A 1L stainless steel polymerization kettle equipped with mechanical stirrer was dried continuously at 130°C for 6h, vacuumized while hot and purged 3 times with N₂. To the polymerization kettle, 500mL of hexane was charged, and 9.6mg (10µmol) of complex 1 was added. The reaction was carried out with stirring vigorously at 70°C for 30min while maintaining an ethylene pressure of 10 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

Example 3
complex 1: complex of formula (II), wherein M is Ni, R₁₁-R₁₅ are H, R₃ is methyl, R₄ is pyridinyl.

A 1L stainless steel polymerization kettle equipped with mechanical stirrer was dried continuously at 130°C for 6h, vacuumized while hot and purged 3 times with N₂. To the polymerization kettle, 500mL of hexane was charged, and 9.6mg (10µmol) of complex 1 was added. The reaction was carried out with stirring vigorously at 100°C for 30min while maintaining an ethylene pressure of 14 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

Example 4
complex 1: complex of formula (II), wherein M is Ni, R₁₁-R₁₅ are H, R₃ is methyl, R₄ is pyridinyl.

A 100mL stainless steel polymerization kettle equipped with mechanical stirrer was dried continuously at 130°C for 6h, vacuumized while hot and purged 3 times with N₂. To the polymerization kettle, 50mL of toluene, 0.9mL (10mmol) of methyl acrylate were charged, and 9.6mg (10µmol) of complex 1 was added. The reaction was carried out with stirring vigorously at 70°C for 60min while maintaining an ethylene pressure of 15 atm. The reaction solution was neutralized with a 10 wt% hydrochloric acid acidified ethanol solution to obtain a polymer. The polymer was dried and weighed for the test of polymerization activity. The test data of weight average molecular weight, molecular weight distribution, polymerization activity and comonomer content of the obtained polymer are shown in Table 1.

Example 5
complex 1: complex of formula (II), wherein M is Ni, R₁₁-R₁₅ are H, R₃ is methyl, R₄ is pyridinyl.

A 100mL stainless steel polymerization kettle equipped with mechanical stirrer was dried continuously at 130°C for 6h, vacuumized while hot and purged 3 times with N₂. To the polymerization kettle, 50mL of toluene, 1.4mL (10mmol) of butyl acrylate were charged, and 9.6mg (10µmol) of complex 1 was added. The reaction was carried out with stirring vigorously at 70°C for 60min while maintaining an ethylene pressure of 15 atm. The reaction solution was neutralized with a 10 wt% hydrochloric acid acidified ethanol solution to obtain a polymer. The polymer was dried and weighed for the test of polymerization activity. The test data of weight average molecular weight, molecular weight distribution, polymerization activity and comonomer content of the obtained polymer are shown in Table 1.

Example 6
complex 16: complex of formula (II), wherein M is Ni, R₁₁-R₁₅ are H, R₃ is phenyl, R₄ is PMe₃.

A ligand L₁ was prepared according to the method of Example 1.

Under a nitrogen atmosphere, the ligand L₁ (0.654g, 1mmol) and PMe₃ (1mol/L, 2mL) were dissolved in 20mL of toluene, to which a 30mL toluene solution of Ni(COD)₂ (0.55g, 2mmol) was added dropwise at 0°C. The solution was reacted at room temperature for 16h, heated at 50°C for 2h, and evaporated to remove the solvent. 5mL of toluene was added, the obtained solution was filtered, 100ml of heptane was added, and the obtained solution was freeze-crystallized to obtain complex 16, with a yield of 72%. Elemental analysis test C₆₂H₅₈Ni₂O₂P₄: theoretical calculation value: C, 69.18; H, 5.43; test value: C, 79.02; H, 5.71.

A 1L stainless steel polymerization kettle equipped with mechanical stirrer was dried continuously at 130°C for 6h, vacuumized while hot and purged 3 times with N₂. To the polymerization kettle, 500mL of toluene was charged, and 10.8mg (10µmol) of complex 16 was added. The reaction was carried out with stirring vigorously at 50°C for 20min while maintaining an ethylene pressure of 10 atm, and the obtained product is C4-C24 oligomer.

Example 7
complex 53: complex of formula (II), wherein M is Ni, R₁₁-R₁₅ are H, R₃ is Br, R₄ is tetrahydrofuranyl.

A ligand L₁ was prepared according to the method of Example 1.

Under a nitrogen atmosphere, the ligand L₁ (0.654g, 1mmol) was dissolved in tetrahydrofuran, excess NaH (0.072g, 3mmol) was added, the solution was stirred at room temperature for 10 h, and then NaH was removed by filtration. A solution of (DME)NiBr₂ (0.617g, 2mmol) in tetrahydrofuran was added dropwise, and reacted overnight at room temperature. The solvent was removed by suction, and the product was dissolved in dichloromethane (40mL). The solution was filtered to remove the filter cake, the filtrate was concentrated, and recrystallized with heptane to obtain complex 53, with a yield of 79%. Elemental analysis test C₅₂H₄₆Br₂Ni₂O₄P₂: theoretical calculation value: C, 58.15; H, 4.32; test value: C, 58.01; H, 4.53.

A 1L stainless steel polymerization kettle equipped with mechanical stirrer was dried continuously at 130°C for 6h, vacuumized while hot and purged 3 times with N₂. To the polymerization kettle, 500mL of toluene was charged, and 10.7mg (10µmol) of complex 53 was added. The reaction was carried out with stirring vigorously at 30°C for 20 min while maintaining an ethylene pressure of 10 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

Example 8
complex 53: complex of formula (II), wherein M is Ni, R₁₁-R₁₅ are H, R₃ is Br, R₄ is tetrahydrofuranyl.

A 1L stainless steel polymerization kettle equipped with mechanical stirrer was dried continuously at 130°C for 6h, vacuumized while hot and purged 3 times with N₂. To the polymerization kettle, 500mL of hexane was charged, and 10.7mg (10µmol) of complex 53 was added. The reaction was carried out with stirring vigorously at 80°C for 30min while maintaining an ethylene pressure of 10 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

Example 9
complex 53: complex of formula (II), wherein M is Ni, R₁₁-R₁₅ are H, R₃ is Br, R₄ is tetrahydrofuranyl.

A 100mL stainless steel polymerization kettle equipped with mechanical stirrer was dried continuously at 130°C for 6h, vacuumized while hot and purged 3 times with N₂. To the polymerization kettle, 50mL of toluene, 0.9mL (0.01mol) of methyl acrylate were charged, and 10.7mg (10µmol) of complex 53 was added. The reaction was carried out with stirring vigorously at 70°C for 60min while maintaining an ethylene pressure of 15 atm. The reaction solution was neutralized with a 10 wt% hydrochloric acid acidified ethanol solution to obtain a polymer. The polymer was dried and weighed for the test of polymerization activity. The test data of weight average molecular weight, molecular weight distribution, polymerization activity and comonomer content of the obtained polymer are shown in Table 1.

Example 10
complex 65: complex of formula (II), wherein M is Ni, R₁₂ and R₁₄ are -CF₃, R₁₁, R₁₃ and R₁₅ are H, R₃ is Br, R₄ is tetrahydrofuranyl.

Under a nitrogen atmosphere, a compound of formula (III) (3.74g, 10mmol, R configuration) was dissolved in anhydrous diethyl ether (120mL), an n-butyl lithium solution (2.7M, 11.1mL, 30mmol) was added dropwise at room temperature, and the solution was stirred at room temperature for 4h. Tetrahydrofuran (120mL) was added, and the obtained black solution containing precipitate was further stirred for 1h. Bis(3,5-di(trifluoromethyl)phenyl)phosphine chloride (30mmol, 14.78g) was added at 0°C. The solution was stirred at room temperature for 1h, and then quenched with the addition of NH₄Cl aqueous solution (5mL). The organic phase was extracted with ethyl acetate, and the obtained organic phase was dried over anhydrous sodium sulfate, recrystallized with dichloromethane/hexane, to obtain a white crystalline compound. Methanol (100mL), 5mL of concentrated hydrochloric acid (37wt%) were added, and reacted under reflux for 16h. After the reaction as tracked using TLC was completed, the organic solvent was removed, and the product was dissolved in ethyl acetate, and neutralized with NaHCO₃ aqueous solution. The organic phase was extracted, dried over anhydrous MgSO₄, filtered, concentrated, and separated by column chromatography (dichloromethane as solvent) to obtain ligand L₂, 4.78g, with a yield of 40%. ¹H NMR (400 MHz, DMSO) δ: 9.10 (s, 2H), 8.17 (s, 2H), 8.13 (s, 2H), 8.08 (dd, J = 13.3, 6.5 Hz, 8H), 7.81 (d, J = 7.6 Hz, 2H), 7.56 (d, J = 8.4 Hz, 2H), 7.27 (dd, 4H), 6.90 (d, J = 8.2 Hz, 2H). High resolution mass spectrometry test: theoretical calculation value: 1198.09; test value: 1198.95. ³¹P NMR (162 MHz, DMSO): δ = -8.37 (s). ¹H NMR (400 MHz, CDCl₃) δ 5.14(s, 2H), 7.08-7.11 (m, 2H), 7.41-7.46 (m, 4H), 7.63 (d, 2H), 7.78-7.81(m, 2H), 7.84(d, 4H), 7.86(d, 4H), 7.91(s, 2H), 7.94(s, 2H).

Under a nitrogen atmosphere, the ligand L₂ (1.20g, 1mmol) was dissolved in tetrahydrofuran, excess NaH (0.072g, 3mmol) was added, the solution was stirred at room temperature for 10 h, and then NaH was removed by filtration. A solution of (DME)NiBr₂ (0.617g, 2mmol) in tetrahydrofuran was added dropwise, and reacted overnight at room temperature. The solvent was removed by suction, and the product was dissolved in dichloromethane (40mL). The solution was filtered to remove the filter cake, the filtrate was concentrated, and recrystallized with heptane to obtain complex 65, with a yield of 76%. Elemental analysis test C₆₀H₃₈Br₂F₂₄Ni₂O₄P₂: theoretical calculation value: C, 44.54; H, 2.37; test value: C, 44.31; H, 2.51.

A 1L stainless steel polymerization kettle equipped with mechanical stirrer was dried continuously at 130°C for 6h, vacuumized while hot and purged 3 times with N₂. To the polymerization kettle, 500mL of toluene was charged, and 8.1mg (5µmol) of complex 65 was added. The reaction was carried out with stirring vigorously at 30°C for 20min while maintaining an ethylene pressure of 10 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

Example 11
complex 65: complex of formula (II), wherein M is Ni, R₁₂ and R₁₄ are -CF₃, R₁₁, R₁₃ and R₁₅ are H, R₃ is Br, R₄ is tetrahydrofuranyl.

A 1L stainless steel polymerization kettle equipped with mechanical stirrer was dried continuously at 130°C for 6h, vacuumized while hot and purged 3 times with N₂. To the polymerization kettle, 500mL of hexane was charged, and 8.1mg (5µmol) of complex 65 was added. The reaction was carried out with stirring vigorously at 80°C for 30min while maintaining an ethylene pressure of 10 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

Example 12
complex 65: complex of formula (II), wherein M is Ni, R₁₂ and R₁₄ are -CF₃, R₁₁, R₁₃ and R₁₅ are H, R₃ is Br, R₄ is tetrahydrofuranyl.

A 100mL stainless steel polymerization kettle equipped with mechanical stirrer was dried continuously at 130°C for 6h, vacuumized while hot and purged 3 times with N₂. To the polymerization kettle, 50mL of toluene, 0.9mL (10mmol) of methyl acrylate were charged, and 8.1mg (5µmol) of complex 65 was added. The reaction was carried out with stirring vigorously at 50°C for 60min while maintaining an ethylene pressure of 15 atm. The reaction solution was neutralized with a 10 wt% hydrochloric acid acidified ethanol solution to obtain a polymer. The polymer was dried and weighed for the test of polymerization activity. The test data of weight average molecular weight, molecular weight distribution, polymerization activity and comonomer content of the obtained polymer are shown in Table 1.

Example 13
complex 11: complex of formula (II), wherein M is Ni, R₁₂ and R₁₄ are methyl, R₁₁, R₁₃ and R₁₅ are H, R₃ is methyl, R₄ is pyridinyl.

Its synthesis steps were similar to those of complex 1 in the above-mentioned Example 1, except for replacing diphenylphosphine chloride with di(3,5-dimethyl phenyl)phosphine chloride.

Under a nitrogen atmosphere, a compound of formula (III) (3.74g, 10mmol, R configuration) was dissolved in anhydrous diethyl ether (120mL), an n-butyl lithium solution (2.5M, 12mL, 30mmol) was added dropwise at room temperature, and the solution was stirred at room temperature for 4h. Tetrahydrofuran (120mL) was added, and the obtained black solution containing precipitate was further stirred for 1h. Di(3,5-dimethyl phenyl)phosphine chloride (30mmol, 8.30g) was added at 0°C. The solution was stirred at room temperature for 1h, and then quenched with the addition of NH₄Cl aqueous solution (5mL). The organic phase was extracted with ethyl acetate, and the obtained organic phase was dried over anhydrous sodium sulfate, recrystallized with dichloromethane/hexane, to obtain a white crystalline compound. Methanol (100mL), 5mL of concentrated hydrochloric acid (37wt%) were added, and reacted under reflux for 16h. After the reaction as tracked using TLC was completed, the organic solvent was removed, and the product was dissolved in ethyl acetate, and neutralized with NaHCO₃ aqueous solution. The organic phase was extracted, dried over anhydrous MgSO₄, filtered, concentrated, and separated by column chromatography (dichloromethane as solvent) to obtain ligand L₃, with a yield of 58%. ¹H NMR (400 MHz, CDCl₃) δ: 2.28 (s, 24H), 5.43 (s, 2H), 6.96-7.05 (m, 12H), 7.17 (m, 2H), 7.24-7.29 (m, 4H), 7.43 (d, 2H), 7.65 (m, 2H). High resolution mass spectrometry test: theoretical calculation value: 766.31; test value: 767.32. ³¹P NMR (162 MHz, CDCl₃) δ = -15.4 (s).

Under a nitrogen atmosphere, the ligand L₃ (0.77g, 1mmol) was dissolved in tetrahydrofuran, excess NaH (0.072g, 3mmol) was added, the solution was stirred at room temperature for 10 h, and then NaH was removed by filtration. A solution of (DME)NiBr₂ (0.617g, 2mmol) in tetrahydrofuran was added dropwise, and reacted overnight at room temperature. The solvent was removed by suction, and the product was dissolved in dichloromethane (40mL). The solution was filtered to remove the filter cake, the filtrate was concentrated, and recrystallized with heptane to obtain complex 11, with a yield of 76%. Elemental analysis test C₆ₒH₆₂Br₂Ni₂O₄P₂: theoretical calculation value: C, 60.75; H, 5.27; test value: C, 60.37; H, 5.51.

A 1L stainless steel polymerization kettle equipped with mechanical stirrer was dried continuously at 130°C for 6h, vacuumized while hot and purged 3 times with N₂. To the polymerization kettle, 500mL of toluene was charged, and 11.8mg (10µmol) of complex 11 was added. The reaction was carried out with stirring vigorously at 50°C for 20min while maintaining an ethylene pressure of 10 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

Example 14
complex 68: complex of formula (II), wherein M is Pd, R₁₁-R₁₅ are H, R₃ is methyl, R₄ is pyridinyl.

A ligand L₁ was prepared according to the method of Example 1.

Under a nitrogen atmosphere, the ligand L₁ (0.65g, 1mmol) was dissolved in toluene (20mL), and dropped into toluene (15mL) solution containing a metal palladium source (TMEDA)PdMe₂ (0.51g, 2 mmol). Then, the solution of the ligand was added dropwise to the solution of the metal palladium source, the obtained solution was reacted with stirring vigorously at room temperature for 5h, 0.6mL of pyridine was added, the obtained solution was reacted overnight, and filtered to obtain a black solution. The solvent was removed under vacuum to obtain a palladium complex 68, with a yield of 84 %. ¹H NMR (400 MHz, C6D6) δ: 8.92(m, 4H), 7.79 (m, 6H,), 7.33 (m, 2H), 7.07-7.18 (m, 22H), 6.94-7.00 (m, 4H), 6.81-6.86(m, 2H), 0.73(d, 6H). Elemental analysis test C₅₆H₄₆N₂O₂P₂Pd₂: theoretical calculation value: C, 63.83; H, 4.40; N, 2.66; test value: C, 63.62; H, 4.60; N, 2.73.

A 100mL glass polymerization tube equipped with mechanical stirrer was dried continuously at 130°C for 6h, vacuumized while hot and purged 3 times with N₂. To the polymerization kettle, 5.0mL of toluene, 5.0mL of norbornene were charged, and 4.2mg (4µmol) of complex 68 was added. 40µmol of B(C₆F₅)₃ was added, and the reaction was carried out with stirring vigorously at 60°C for 10min. The reaction solution was neutralized with a 10 wt% hydrochloric acid acidified ethanol solution to obtain polynorbornene. The polymer was dried and weighed for the test of polymerization activity. The test data of weight average molecular weight, molecular weight distribution and polymerization activity of the obtained polymer are shown in Table 1.

Example 15
complex 62: complex of formula (II), wherein M is Ni, R₁₃ is methoxy, R₁₁, R₁₂, R₁₄ and R₁₅ are H, R₃ is Br, R₄ is tetrahydrofuranyl.

Under a nitrogen atmosphere, a compound of formula (III) (3.74g, 10mmol, S configuration) was dissolved in anhydrous diethyl ether (120mL), an n-butyl lithium solution (2.7M, 11.1mL, 30mmol) in hexane was added dropwise at room temperature condition, and the solution was stirred at room temperature for 4h. Tetrahydrofuran (120mL) was added, and the obtained black solution containing precipitate was further stirred for 1h. Di(4-methoxy)phosphine chloride (30mmol, 8.42g) was added at 0°C. The solution was stirred at room temperature for 1h, and then quenched with the addition of NH₄Cl aqueous solution (5mL). The organic phase was extracted with ethyl acetate, and the obtained organic phase was dried over anhydrous sodium sulfate, recrystallized with dichloromethane/hexane, to obtain a white crystalline compound. Methanol (100mL), 5mL of concentrated hydrochloric acid (37wt%) were added, and reacted under reflux for 16h. After the reaction as tracked using TLC was completed, the organic solvent was removed, and the compound was dissolved in ethyl acetate, and neutralized with NaHCO₃ aqueous solution. The organic phase was extracted, dried over anhydrous MgSO₄, filtered, concentrated, and separated by column chromatography (dichloromethane as solvent) to obtain ligand L₄, with a yield of 56 %. Elemental analysis test C₄₈H₄₀O₆P₂. theoretical calculation value: C, 74.41; H, 5.20; test value: C, 74.32; H, 5.38.

Under a nitrogen atmosphere, the ligand L₄ (0.77g, 1mmol) was dissolved in tetrahydrofuran, excess NaH (0.072g, 3mmol) was added, the solution was stirred at room temperature for 10 h, and then NaH was removed by filtration. A solution of (DME)NiBr₂ (0.617g, 2mmol) in tetrahydrofuran was added dropwise, and reacted overnight at room temperature. The solvent was removed by suction, and the product was dissolved in dichloromethane (40mL). The solution was filtered to remove the filter cake, the filtrate was concentrated, and recrystallized with heptane to obtain complex 62, with a yield of 77%. Elemental analysis test C₅₆H₅₄Br₂Ni₂O₈P₂: theoretical calculation value: C, 56.32; H, 4.56; test value: C, 56.31; H, 5.11.

A 1L stainless steel polymerization kettle equipped with mechanical stirrer was dried continuously at 130°C for 6h, vacuumized while hot and purged 3 times with N₂. To the polymerization kettle, 500mL of toluene was charged, and 6.0mg (5µmol) of complex 62 was added. The reaction was carried out with stirring vigorously at 30°C for 20min while maintaining an ethylene pressure of 10 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

### Comparative Example 1

10atm ethylene: A 1L stainless steel polymerization kettle equipped with mechanical stirrer was dried continuously at 130°C for 6h, vacuumized while hot and purged 3 times with N₂. To the polymerization kettle, 500mL of hexane was charged, 5.0ml methylaluminoxane (MAO) (1.53mol/L toluene solution), and 10.1mg (20µmol) of complex A (as to its synthesis process, please refer to the literature Acta Agron. Sin. 2012, 29, 1381; ACS Catalysis 2021, 11, 5, 2902-2911) was added. The reaction was carried out with stirring vigorously at 70°C for 30min while maintaining an ethylene pressure of 10 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

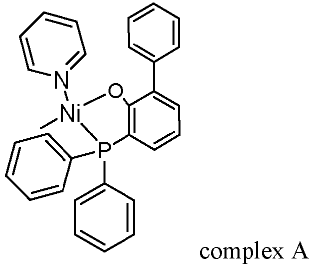

### Comparative Example 2

A 100mL stainless steel polymerization kettle equipped with mechanical stirrer was dried continuously at 130°C for 6h, vacuumized while hot and purged 3 times with N₂. To the polymerization kettle, 50mL of toluene, 0.9mL (10.0mmol) of methyl acrylate were charged, and 10.1mg (10µmol) of complex A was added. The reaction was carried out with stirring vigorously at 70°C for 60min while maintaining an ethylene pressure of 15 atm. The reaction solution was neutralized with a 10 wt% hydrochloric acid acidified ethanol solution to obtain a polymer. The polymer was dried and weighed for the test of polymerization activity. The test data of weight average molecular weight, molecular weight distribution, polymerization activity and comonomer content of the obtained polymer are shown in Table 1.

### Comparative Example 3

A 100mL glass polymerization tube equipped with mechanical stirrer was dried continuously at 130°C for 6h, vacuumized while hot and purged 3 times with N₂. To the polymerization tube, 5.0mL of toluene, 5.0mL of norbornene were charged, and 4.3mg (8µmol) of complex B (as to its synthesis process, please refer to the literature Appl Organometal Chem. 2017; e4013) was added. 40µmol of B(C₆F₅)₃ was added, and the reaction was carried out with stirring vigorously at 60°C for 10min. The reaction solution was neutralized with a 10 wt% hydrochloric acid acidified ethanol solution to obtain polynorbornene. The polymer was dried and weighed for the test of polymerization activity. The test data of weight average molecular weight, molecular weight distribution and polymerization activity of the obtained polymer are shown in Table 1.

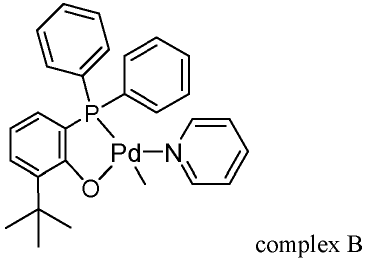

**Table 1**

| Example | Complex | Polymerization activity (g/molcat·h) | *M*_{w}×10⁻⁴ | *M_{w}*/*Mₙ* | Comonomer content (mol%) |
|---|---|---|---|---|---|
| Example 1 | complex 1 | 4.8×10⁵ | 5.8 | 2.0 | - |
| Example 2 | complex 1 | 9.2×10⁶ | 3.1 | 2.0 | - |
| Example 3 | complex 1 | 5.2×10⁶ | 2.1 | 2.1 | - |
| Example 4 | complex 1 | 4.2×10⁵ | 3.4 | 2.0 | 5.1 |
| Example 5 | complex 1 | 3.9×10⁵ | 3.4 | 2.0 | 5.3 |
| Example 6 | complex 16 | Oligomerization | - | - | - |
| Example 7 | complex 53 | 8.7×10⁶ | 33.2 | 2.0 | - |
| Example 8 | complex 53 | 4.1×10⁶ | 9.3 | 2.3 | - |
| Example 9 | complex 53 | 4.2×10⁵ | 8.9 | 2.1 | 5.6 |
| Example 10 | complex 65 | 8.9×10⁶ | 27.6 | 2.1 | - |
| Example 11 | complex 65 | 2.8×10⁶ | 3.2 | 2.2 | - |
| Example 12 | complex 65 | 3.2×10⁵ | 11.1 | 2.1 | 5.8 |
| Example 13 | complex 11 | 6.2×10⁶ | 20.2 | 2.2 | - |
| Example 14 | complex 68 | 3.2×10⁶ | 16.1 | 1.1 | - |
| Example 15 | complex 62 | 8.4×10⁶ | 22.6 | 2.2 | - |
| Comparative Example 1 | complex A | 2.2×10⁶ | 1.1 | 2.5 | - |
| Comparative Example 2 | complex A | 2.1×10⁴ | 0.11 | 2.0 | 4.4 |
| Comparative Example 3 | complex B | 8.0×10⁵ | 9.0 | 1.3 | - |

As can be seen from the data in Table 1, under similar polymerization conditions, by using the metal complex of the present invention as an olefin polymerization catalyst, a higher homopolymerization/copolymerization activity can be achieved, and the obtained polymer has an obviously higher molecular weight and a narrower molecular weight distribution.

The preferred embodiments of the present invention have been described above in detail, but the present invention is not limited thereto. Within the scope of the technical idea of the present invention, many simple modifications can be made to the technical solution of the present invention, including various technical features being combined in any other suitable way, and these simple modifications and combinations should also be regarded as the disclosure of the present invention, and all fall within the scope of the present invention.

## Claims

1. A phosphine-phenol late transition metal complex, **characterized in that** it has a structural formula shown in formula (I), 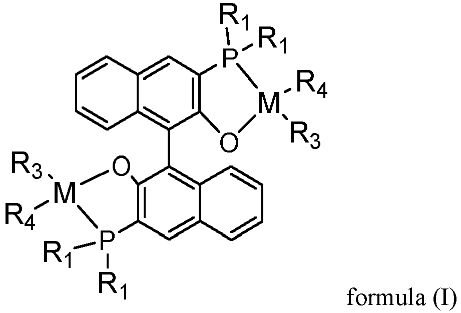
wherein, M is selected from Group VIII metals, R₁ is selected from substituted or unsubstituted C6-C20 aryl, R₃ and R₄ are each independently selected from halogen, C1-C10 hydrocarbyl, P(R₅)₃, NR₆R₇ and OR₈R₉, or R₃ and R₄ are interconnected to form an eight-membered ring, wherein R₅ is selected from substituted or unsubstituted C1-C10 alkyl and substituted or unsubstituted C6-C10 aryl, R₆, R₇, R₈ and R₉ are each independently selected from C1-C10 hydrocarbyl, or R₆, R₇ and N are interconnected to form a five- or six-membered ring, or R₈, R₉ and O are interconnected to form a five- or six-membered ring,
preferably, C6-C20 aryl is selected from phenyl, 4-methyl phenyl, 4-ethyl phenyl, dimethyl phenyl, vinyl phenyl, anthryl, naphthyl, or biphenyl;
preferably, for substituted C6-C20 aryl, the substituent thereof is selected from H, halogen, hydroxy, substituted or unsubstituted alkoxy, substituted or unsubstituted C1-C20 hydrocarbyl; preferably, alkoxy is C1-C6 alkoxy;
preferably, for substituted alkoxy and substituted C1-C20 hydrocarbyl, the substituents thereof are selected from halogen, hydroxy, C1-C6 alkyl, halo-substituted C1-C6 alkyl, C1-C6 alkoxy and halo-substituted C1-C6 alkoxy;
preferably, C1-C10 hydrocarbyl is selected from C1-C8 hydrocarbyl; and/or
preferably, C1-C20 hydrocarbyl is selected from C1-C10 alkyl or C6-C15 aryl.

2. The phosphine-phenol late transition metal complex as claimed in claim 1, **characterized in that** it has a structural formula shown in formula (II), 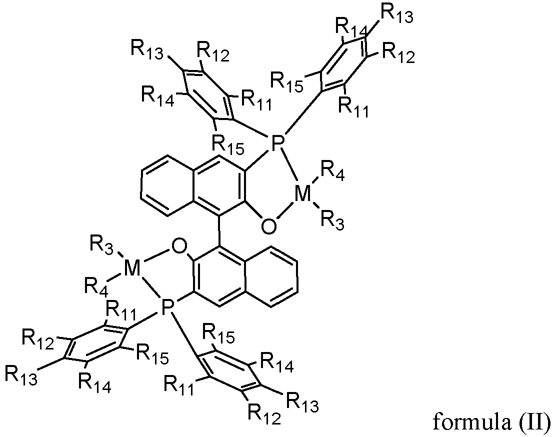 wherein, R₁₁-R₁₅ are each independently selected from H, halogen, hydroxy, substituted or unsubstituted alkoxy, substituted or unsubstituted C1-C20 hydrocarbyl; preferably, for substituted alkoxy and substituted C1-C20 hydrocarbyl, the substituents thereof are selected from halogen, hydroxy, C1-C6 alkyl, halo-substituted C1-C6 alkyl, C1-C6 alkoxy and halo-substituted C1-C6 alkoxy.

3. The phosphine-phenol late transition metal complex as claimed in claim 1 or 2, **characterized in that**, M is selected from nickel and palladium.

4. The phosphine-phenol late transition metal complex as claimed in any of claims 1-3, **characterized in that**, R₁₁-R₁₅ are each independently selected from H, halogen, hydroxy, substituted or unsubstituted alkoxy or substituted or unsubstituted C1-C10 alkyl and substituted or unsubstituted C6-C15 aryl;
preferably, at least one of R₃ and R₄ is selected from halogen and C1-C8 hydrocarbyl, or R₃ and R₄ are interconnected to form an eight-membered ring;
preferably, for substituted alkoxy, substituted C1-C10 alkyl and substituted C6-C15 aryl, the substituents are independently selected from halogen, hydroxy, C1-C6 alkyl, halo-substituted C1-C6 alkyl, C1-C6 alkoxy and halo-substituted C1-C6 alkoxy;
preferably, the C1-C6 alkyl is selected from methyl, ethyl, n-propyl, isopropyl, n-butyl, isobutyl, n-pentyl, isopentyl, n-hexyl, isohexyl and 3,3-dimethyl butyl;
preferably, the C1-C6 alkoxy is selected from methoxy, ethoxy, n-propoxy, isopropoxy, n-butoxy, isobutoxy, n-pentyloxy, isopentyloxy, n-hexyloxy, isohexyloxy and 3,3-dimethyl butoxy;
preferably, the halogen is selected from fluorine, chlorine, bromine and iodine.

5. The phosphine-phenol late transition metal complex as claimed in any of claims 1-4, **characterized in that** it is selected from a group consisting of the following complexes,
complex 1: complex of formula (II), wherein M is Ni, R₁₁-R₁₅ are H, R₃ is methyl, R₄ is pyridinyl;
complex 2: complex of formula (II), wherein M is Ni, R₁₁-R₁₅ are F, R₃ is methyl, R₄ is pyridinyl;
complex 3: complex of formula (II), wherein M is Ni, R₁₁ is phenyl, R₁₂-R₁₅ are H, R₃ is methyl, R₄ is pyridinyl;
complex 4: complex of formula (II), wherein M is Ni, R₁₁ is methyl, R₁₂-R₁₅ are H, R₃ is methyl, R₄ is pyridinyl;
complex 5: complex of formula (II), wherein M is Ni, R₁₃ is methyl, R₁₁, R₁₂, R₁₄ and R₁₅ are H, R₃ is methyl, R₄ is pyridinyl;
complex 6: complex of formula (II), wherein M is Ni, R₁₃ is -CF₃, R₁₁, R₁₂, R₁₄ and R₁₅ are H, R₃ is methyl, R₄ is pyridinyl;
complex 7: complex of formula (II), wherein M is Ni, R₁₃ is F, R₁₁, R₁₂, R₁₄ and R₁₅ are H, R₃ is methyl, R₄ is pyridinyl;
complex 8: complex of formula (II), wherein M is Ni, R₁₃ is Cl, R₁₁, R₁₂, R₁₄ and R₁₅ are H, R₃ is methyl, R₄ is pyridinyl;
complex 9: complex of formula (II), wherein M is Ni, R₁₁, R₁₃ and R₁₅ are methyl, R₁₂ and R₁₄ are H, R₃ is methyl, R₄ is pyridinyl;
complex 10: complex of formula (II), wherein M is Ni, R₁₃ is methoxy, R₁₁, R₁₂, R₁₄ and R₁₅ are H, R₃ is methyl, R₄ is pyridinyl;
complex 11: complex of formula (II), wherein M is Ni, R₁₂ and R₁₄ are methyl, R₁₁, R₁₃ and R₁₅ are H, R₃ is methyl, R₄ is pyridinyl;
complex 12: complex of formula (II), wherein M is Ni, R₁₂ and R₁₄ are methyl, R₁₃ is methoxy, R₁₁ and R₁₅ are H, R₃ is methyl, R₄ is pyridinyl;
complex 13: complex of formula (II), wherein M is Ni, R₁₂ and R₁₄ are -CF₃, R₁₁, R₁₃ and R₁₅ are H, R₃ is methyl, R₄ is pyridinyl;
complex 14: complex of formula (II), wherein M is Ni, R₁₁ and R₁₅ are methoxy, R₁₂-R₁₄ are H, R₃ is methyl, R₄ is pyridinyl;
complex 15: complex of formula (II), wherein M is Ni, R₁₁ is methoxy, R₁₂-R₁₅ are H, R₃ is methyl, R₄ is pyridinyl;
complex 16: complex of formula (II), wherein M is Ni, R₁₁-R₁₅ are H, R₃ is phenyl, R₄ is PMe₃;
complex 17: complex of formula (II), wherein M is Ni, R₁₁ and R₁₅ are F, R₃ is phenyl, R₄ is PMe₃;
complex 18: complex of formula (II), wherein M is Ni, R₁₁ is phenyl, R₁₂-R₁₅ are H, R₃ is phenyl, R₄ is PMe₃;
complex 19: complex of formula (II), wherein M is Ni, R₁₁ is methyl, R₁₂-R₁₅ are H, R₃ is phenyl, R₄ is PMe₃;
complex 20: complex of formula (II), wherein M is Ni, R₁₃ is methyl, R₁₁, R₁₂, R₁₄ and R₁₅ are H, R₃ is phenyl, R₄ is PMe₃;
complex 21: complex of formula (II), wherein M is Ni, R₁₃ is -CF₃, R₁₁, R₁₂, R₁₄ and R₁₅ are H, R₃ is phenyl, R₄ is PMe₃;
complex 22: complex of formula (II), wherein M is Ni, R₁₃ is F, R₁₁, R₁₂, R₁₄ and R₁₅ are H, R₃ is phenyl, R₄ is PMe₃;
complex 23: complex of formula (II), wherein M is Ni, R₁₃ is Cl, R₁₁, R₁₂, R₁₄ and R₁₅ are H, R₃ is phenyl, R₄ is PMe₃;
complex 24: complex of formula (II), wherein M is Ni, R₁₁, R₁₃ and R₁₅ are methyl, R₁₂ and R₁₄ are H, R₃ is phenyl, R₄ is PMe₃;
complex 25: complex of formula (II), wherein M is Ni, R₁₃ is methoxy, R₁₁, R₁₂, R₁₄ and R₁₅ are H, R₃ is phenyl, R₄ is PMe₃;
complex 26: complex of formula (II), wherein M is Ni, R₁₂ and R₁₄ are methyl, R₁₁, R₁₃ and R₁₅ are H, R₃ is phenyl, R₄ is PMe₃;
complex 27: complex of formula (II), wherein M is Ni, R₁₂ and R₁₄ are methyl, R₁₃ is methoxy, R₁₁ and R₁₅ are H, R₃ is phenyl, R₄ is PMe₃;
complex 28: complex of formula (II), wherein M is Ni, R₁₂ and R₁₄ are -CF₃, R₁₁, R₁₃ and R₁₅ are H, R₃ is phenyl, R₄ is PMe₃;
complex 29: complex of formula (II), wherein M is Ni, R₁₁ and R₁₅ are methoxy, R₁₂-R₁₄ are H, R₃ is phenyl, R₄ is PMe₃;
complex 30: complex of formula (II), wherein M is Ni, R₁₁ is methoxy, R₁₂-R₁₅ are H, R₃ is phenyl, R₄ is PMe₃;
complex 31: complex of formula (II), wherein M is Ni, R₁₁-R₁₅ are H, R₃, R₄ are interconnected to form cyclooctenyl;
complex 32: complex of formula (II), wherein M is Ni, R₁₁ is phenyl, R₁₂-R₁₅ are H, R₃, R₄ are interconnected to form cyclooctenyl;
complex 33: complex of formula (II), wherein M is Ni, R₁₁ is phenyl, R₁₂-R₁₅ are H, R₃, R₄ are interconnected to form cyclooctenyl;
complex 34: complex of formula (II), wherein M is Ni, R₁₃ is methoxy, R₁₁, R₁₂, R₁₄ and R₁₅ are H, R₃, R₄ are interconnected to form cyclooctenyl;
complex 35: complex of formula (II), wherein M is Ni, R₁₂ and R₁₄ are -CF₃, R₁₁, R₁₃ and R₁₅ are H, R₃, R₄ are interconnected to form cyclooctenyl;
complex 36: complex of formula (II), wherein M is Ni, R₁₁ and R₁₅ are methoxy, R₁₂-R₁₄ are H, R₃, R₄ are interconnected to form cyclooctenyl;
complex 37: complex of formula (II), wherein M is Ni, R₁₁ is methoxy, R₁₂-R₁₅ are H, R₃, R₄ are interconnected to form cyclooctenyl;
complex 38: complex of formula (II), wherein M is Ni, R₁₁-R₁₅ are H, R₃ is Cl, R₄ is tetrahydrofuranyl;
complex 39: complex of formula (II), wherein M is Ni, R₁₁-R₁₅ are F, R₃ is Cl, R₄ is tetrahydrofuranyl;
complex 40: complex of formula (II), wherein M is Ni, R₁₁ is phenyl, R₁₂-R₁₅ are H, R₃ is Cl, R₄ is tetrahydrofuranyl;
complex 41: complex of formula (II), wherein M is Ni, R₁₁ is methyl, R₁₂-R₁₅ are H, R₃ is Cl, R₄ is tetrahydrofuranyl;
complex 42: complex of formula (II), wherein M is Ni, R₁₃ is methyl, R₁₁, R₁₂, R₁₄ and R₁₅ are H, R₃ is Cl, R₄ is tetrahydrofuranyl;
complex 43: complex of formula (II), wherein M is Ni, R₁₃ is -CF₃, R₁₁, R₁₂, R₁₄ and R₁₅ are H, R₃ is Cl, R₄ is tetrahydrofuranyl;
complex 44: complex of formula (II), wherein M is Ni, R₁₃ is F, R₁₁, R₁₂, R₁₄ and R₁₅ are H, R₃ is Cl, R₄ is tetrahydrofuranyl;
complex 45: complex of formula (II), wherein M is Ni, R₁₃ is Cl, R₁₁, R₁₂, R₁₄ and R₁₅ are H, R₃ is Cl, R₄ is tetrahydrofuranyl;
complex 46: complex of formula (II), wherein M is Ni, R₁₁, R₁₃ and R₁₅ are methyl, R₁₂ and R₁₄ are H, R₃ is Cl, R₄ is tetrahydrofuranyl;
complex 47: complex of formula (II), wherein M is Ni, R₁₃ is methoxy, R₁₁, R₁₂, R₁₄ and R₁₅ are H, R₃ is Cl, R₄ is tetrahydrofuranyl;
complex 48: complex of formula (II), wherein M is Ni, R₁₂ and R₁₄ are methyl, R₁₁, R₁₃ and R₁₅ are H, R₃ is Cl, R₄ is tetrahydrofuranyl;
complex 49: complex of formula (II), wherein M is Ni, R₁₂ and R₁₄ are methyl, R₁₃ is methoxy, R₁₁ and R₁₅ are H, R₃ is Cl, R₄ is tetrahydrofuranyl;
complex 50: complex of formula (II), wherein M is Ni, R₁₂ and R₁₄ are -CF₃, R₁₁, R₁₃ and R₁₅ are H, R₃ is Cl, R₄ is tetrahydrofuranyl;
complex 51: complex of formula (II), wherein M is Ni, R₁₁ and R₁₅ are methoxy, R₁₂-R₁₄ are H, R₃ is Cl, R₄ is tetrahydrofuranyl;
complex 52: complex of formula (II), wherein M is Ni, R₁₁ is methoxy, R₁₂-R₁₅ are H, R₃ is Cl, R₄ is tetrahydrofuranyl;
complex 53: complex of formula (II), wherein M is Ni, R₁₁-R₁₅ are H, R₃ is Br, R₄ is tetrahydrofuranyl;
complex 54: complex of formula (II), wherein M is Ni, R₁₁-R₁₅ are F, R₃ is Br, R₄ is tetrahydrofuranyl;
complex 55: complex of formula (II), wherein M is Ni, R₁₁ is phenyl, R₁₂-R₁₅ are H, R₃ is Br, R₄ is tetrahydrofuranyl;
complex 56: complex of formula (II), wherein M is Ni, R₁₁ is methyl, R₁₂-R₁₅ are H, R₃ is Br, R₄ is tetrahydrofuranyl;
complex 57: complex of formula (II), wherein M is Ni, R₁₃ is methyl, R₁₁, R₁₂, R₁₄ and R₁₅ are H, R₃ is Br, R₄ is tetrahydrofuranyl;
complex 58: complex of formula (II), wherein M is Ni, R₁₃ is -CF₃, R₁₁, R₁₂, R₁₄ and R₁₅ are H, R₃ is Br, R₄ is tetrahydrofuranyl;
complex 59: complex of formula (II), wherein M is Ni, R₁₃ is F, R₁₁, R₁₂, R₁₄ and R₁₅ are H, R₃ is Br, R₄ is tetrahydrofuranyl;
complex 60: complex of formula (II), wherein M is Ni, R₁₃ is Cl, R₁₁, R₁₂, R₁₄ and R₁₅ are H, R₃ is Br, R₄ is tetrahydrofuranyl;
complex 61: complex of formula (II), wherein M is Ni, R₁₁, R₁₃ and R₁₅ are methyl, R₁₂ and R₁₄ are H, R₃ is Br, R₄ is tetrahydrofuranyl;
complex 62: complex of formula (II), wherein M is Ni, R₁₃ is methoxy, R₁₁, R₁₂, R₁₄ and R₁₅ are H, R₃ is Br, R₄ is tetrahydrofuranyl;
complex 63: complex of formula (II), wherein M is Ni, R₁₂ and R₁₄ are methyl, R₁₁, R₁₃ and R₁₅ are H, R₃ is Br, R₄ is tetrahydrofuranyl;
complex 64: complex of formula (II), wherein M is Ni, R₁₂ and R₁₄ are methyl, R₁₃ is methoxy, R₁₁ and R₁₅ are H, R₃ is Br, R₄ is tetrahydrofuranyl;
complex 65: complex of formula (II), wherein M is Ni, R₁₂ and R₁₄ are -CF₃, R₁₁, R₁₃ and R₁₅ are H, R₃ is Br, R₄ is tetrahydrofuranyl;
complex 66: complex of formula (II), wherein M is Ni, R₁₁ and R₁₅ are methoxy, R₁₂-R₁₄ are H, R₃ is Br, R₄ is tetrahydrofuranyl;
complex 67: complex of formula (II), wherein M is Ni, R₁₁ is methoxy, R₁₂-R₁₅ are H, R₃ is Br, R₄ is tetrahydrofuranyl;
complex 68: complex of formula (II), wherein M is Pd, R₁₁-R₁₅ are H, R₃ is methyl, R₄ is pyridinyl;
complex 69: complex of formula (II), wherein M is Pd, R₁₁-R₁₅ are F, R₃ is methyl, R₄ is pyridinyl;
complex 70: complex of formula (II), wherein M is Pd, R₁₁ is phenyl, R₁₂-R₁₅ are H, R₃ is methyl, R₄ is pyridinyl;
complex 71: complex of formula (II), wherein M is Pd, R₁₁ is phenyl, R₁₂-R₁₅ are H, R₃ is methyl, R₄ is pyridinyl;
complex 72: complex of formula (II), wherein M is Pd, R₁₁ is methyl, R₁₂-R₁₅ are H, R₃ is methyl, R₄ is pyridinyl;
complex 73: complex of formula (II), wherein M is Pd, R₁₃ is methyl, R₁₁, R₁₂, R₁₄ and R₁₅ are H, R₃ is methyl, R₄ is pyridinyl;
complex 74: complex of formula (II), wherein M is Pd, R₁₃ is -CF₃, R₁₁, R₁₂, R₁₄ and R₁₅ are H, R₃ is methyl, R₄ is pyridinyl;
complex 75: complex of formula (II), wherein M is Pd, R₁₃ is F, R₁₁, R₁₂, R₁₄ and R₁₅ are H, R₃ is methyl, R₄ is pyridinyl;
complex 76: complex of formula (II), wherein M is Pd, R₁₃ is Cl, R₁₁, R₁₂, R₁₄ and R₁₅ are H, R₃ is methyl, R₄ is pyridinyl;
complex 77: complex of formula (II), wherein M is Pd, R₁₁, R₁₃ and R₁₅ are methyl, R₁₂ and R₁₄ are H, R₃ is methyl, R₄ is pyridinyl;
complex 78: complex of formula (II), wherein M is Pd, R₁₃ is methoxy, R₁₁, R₁₂, R₁₄ and R₁₅ are H, R₃ is methyl, R₄ is pyridinyl;
complex 79: complex of formula (II), wherein M is Pd, R₁₂ and R₁₄ are methyl, R₁₁, R₁₃ and R₁₅ are H, R₃ is methyl, R₄ is pyridinyl;
complex 80: complex of formula (II), wherein M is Pd, R₁₂ and R₁₄ are methyl, R₁₃ is methoxy, R₁₁ and R₁₅ are H, R₃ is methyl, R₄ is pyridinyl;
complex 81: complex of formula (II), wherein M is Pd, R₁₂ and R₁₄ are -CF₃, R₁₁, R₁₃ and R₁₅ are H, R₃ is methyl, R₄ is pyridinyl;
complex 82: complex of formula (II), wherein M is Pd, R₁₁ and R₁₅ are methoxy, R₁₂-R₁₄ are H, R₃ is methyl, R₄ is pyridinyl;
complex 83: complex of formula (II), wherein M is Pd, R₁₁ is methoxy, R₁₂-R₁₅ are H, R₃ is methyl, R₄ is pyridinyl;
complex 84: complex of formula (II), wherein M is Pd, R₁₁-R₁₅ are H, R₃ is methyl, R₄ is Cl;
complex 85: complex of formula (II), wherein M is Pd, R₁₁ is phenyl, R₁₂-R₁₅ are H, R₃ is methyl, R₄ is Cl;
complex 86: complex of formula (II), wherein M is Pd, R₁₃ is methoxy, R₁₁, R₁₂, R₁₄ and R₁₅ are H, R₃ is methyl, R₄ is Cl;
complex 87: complex of formula (II), wherein M is Pd, R₁₂ and R₁₄ are -CF₃, R₁₁, R₁₃ and R₁₅ are H, R₃ is methyl, R₄ is Cl;
complex 88: complex of formula (II), wherein M is Pd, R₁₁ and R₁₅ are methoxy, R₁₂-R₁₄ are H, R₃ is methyl, R₄ is Cl;
complex 89: complex of formula (II), wherein M is Pd, R₁₁ is methoxy, R₁₂-R₁₅ are H, R₃ is methyl, R₄ is Cl.

6. A method for preparing the phosphine-phenol late transition metal complex as claimed in any of claims 1-5, **characterized in that** the method comprises:
(1) reacting a compound of formula (III) with a compound of formula (IV), to obtain a ligand;
(2) reacting the ligand with an M metal compound; 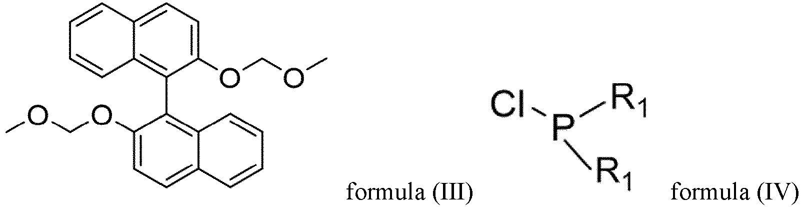 wherein, M metal is selected from Group VIII metals, preferably nickel and/or palladium; the definition of R₁ is the same as that defined in claim 1.

7. The method as claimed in claim 6, **characterized in that**, the M metal compound is selected from at least one of dimethyl dipyridinyl nickel, bis(1,5-cyclooctadienyl) nickel, tetrapyridinyl nickel dichloride, ethylene glycol dimethyl ether nickel bromide, ethylene glycol dimethyl ether nickel chloride, di(trimethylphosphino) nickel dichloride, di(pyridinyl) di[(trimethylsilyl)methyl] nickel, (phenyl)(N,N,N',N'-tetramethyl-1,2-ethylene diamine) nickel chloride, dibenzyl dipyridinyl nickel, phenyl (trimethylphosphino) nickel bromide, phenyl (triethylphosphino) nickel chloride, diphenyl di(trimethylphosphino) nickel, di(trimethylphosphino) nickel dichloride, dimethyl dipyridinyl palladium, dipyridinyl palladium dichloride, di(pyridinyl) di[(trimethylsilyl)methyl] palladium, dibenzyl dipyridinyl palladium and methyl-1,5-cyclooctadiene-palladium chloride.

8. The method as claimed in claim 6 or 7, **characterized in that**, the reaction of step (2) is carried out in the presence of a reaction solvent, the reaction solvent being tetrahydrofuran.

9. Use of the phosphine-phenol late transition metal complex as claimed in claims 1-5 in olefin polymerization.

10. An olefin polymerization method, **characterized in that** it comprises carrying out olefin polymerization reaction in the presence of the phosphine-phenol late transition metal complex as claimed in any of claims 1-5;
preferably, the olefin polymerization reaction has a temperature of -78°C~200°C, preferably -20°C~150°C, and a pressure of 0.01~10MPa, preferably 0.01~5MPa.

11. A method for preparing α-olefin polymer, **characterized in that** it comprises copolymerizing (a) an α-olefin and (b) a (meth)acrylate monomer, a vinyl monomer or an allyl monomer in the presence of the phosphine-phenol late transition metal complex as claimed in any of claims 1-5;
preferably, the (meth)acrylate monomer is represented by the general formula CH₂=C(R₃₁)CO₂(R₃₂), wherein,
preferably, R₃₁ is H or C1-C10 hydrocarbyl, which optionally has a branched, cyclic and/or unsaturated bond;
preferably, R₃₂ is C1-C30 hydrocarbyl, which optionally has a branched, cyclic and/or unsaturated bond, and R₃₂ optionally contains a heteroatom at any position therein.
